# EUROPEAN PATENT SPECIFICATION

(11) **EP 3 505 620 B1**
(45) Date of publication and mention of the grant of the patent: **26.07.2023**
(21) Application number: 17843522.8
(22) Date of filing: 21.08.2017
(51) Int. Cl.: C12N 5/071, C12N 7/00, C12N 15/09

(54) **2D ORGANOID FOR INFECTION AND CULTURE OF HUMAN DIARRHEA VIRUS, AND USE OF SAID 2D ORGANOID**
2D ORGANOID FÜR INFEKTIONEN UND ANZUCHT VON MENSCHLICHEM DIARRHOEVIRUS UND VERWENDUNG DES ZWEIDIMENSIONALEN ORGANOIDS
ORGANOÏDE 2D POUR L'INFECTION ET LA CULTURE DE VIRUS DE LA DIARRHÉE HUMAINE, ET UTILISATION DUDIT ORGANOÏDE 2D

(30) Priority: 24.08.2016 JP 2016163711
(43) Date of publication of application: 03.07.2019
(73) Proprietor: Keio University, Tokyo 108-8345 (JP)
(72) Inventor: SATO, Toshiro, Tokyo 160-8582 (JP); KATAYAMA, Kazuhiko, Tokyo 160-8582 (JP)
(74) Representative: Marks & Clerk LLP
(86) International application number: PCT/JP2017/029743
(87) International publication number: WO 2018/038042

(56) References cited:
- EP-A1- 3 418 376
- WO-A1-2014/132933
- WO-A1-2017/059449
- JP-A- 2012 516 685
- JP-A- 2014 506 801
- JP-A- 2016 506 246
- JP-A- 2016 512 958
- DATE SHOICH et al.: "Mini-Gut Organoids: Reconstitution of the Stem Cell Niche", Annu. Rev. Cell Dev. Biol., vol. 31, 2015, pages 269-289, XP055471729, DOI: doi:10.1146/annurev-cellbio-100814-125218
- SATO TOSHIRO et al.: "Long-term Expansion of Epithelial Organoids From Human Colon, Adenoma, Adenocarcinoma, and Barrett's Epithelium", GASTROENTEROLOGY, vol. 141, 2011, pages 1762-1772, XP028325676, DOI: doi:10.1053/j.gastro.2011.07.050
- MIHARA EMIKO et al.: "Active and water-soluble form of lipidated Wnt protein is maintained by a serum glycoprotein afamin/a-albumin", eLife, vol. 5, February 2016 (2016-02), page ell621, XP055477142, DOI: doi:10.7554/eLife.11621
- ETTAYEBI KHALIL et al.: "Replication of human noroviruses in stem cell -derived human enteroids", Science, vol. 353, no. 6306 25 August 2016 (2016-08-25), pages 1387-1393, XP055471758, DOI: doi:10.1126/science.aaf5080
- SHIMOKAWA MARIKO et al.: "Back to 2D Culture for Ground State of Intestinal Stem Cells", Cell Stem Cell, vol. 17, 2016, pages 5-7, XP055471760, DOI: doi:10.1016/j.stem.2015.06.008
- PAPAFRAGKOU EFSTATHIA et al.: "Challenges of Culturing Human Norovirus in Three-Dimensional Organoid Intestinal Cell Culture Models", PLOS ONE, vol. 8, no. 6, 2013, page e63485, XP055471766, DOI: doi:10.1371/journal.pone.0063485

## Description

### Technical Field

The present invention relates to a 2D organoid for infection and growth culture of human diarrhea virus and use thereof. More specifically, the present invention relates to a production method of human diarrhea virus, a production kit for human diarrhea virus, a culture kit for a 2D organoid for infection and growth culture of human diarrhea virus, and a production method of a 2D organoid for infection and growth culture of human diarrhea virus. Priority is claimed on Japanese Patent Application No. 2016-163711, filed on August 24, 2016.

### Background Art

In the case of being infected with human diarrhea virus such as human norovirus, an antiviral drug having high therapeutic efficacy does not yet exist. Many human diarrhea viruses only infect the intestinal epithelium and grow only therein. Therefore, it is not possible to artificially produce a human diarrhea virus in a large amount in vitro, which is a major problem in the development of therapeutic drugs.

In order to solve the problem, a cell culture system using mouse norovirus has been established (for example, see NPL 1). However, in the cell culture system described in NPL 1, human virus cannot be used. Therefore, the cell culture system described in NPL 1 cannot be applied to research on the human virus and development of a cell culture system of human virus has been expected.

On the other hand, long-term culturing of human intestinal epithelial cells was not possible for a long time. This is considered to be due to an unknown growth factor necessary for maintenance of human intestinal epithelial stem cells. In recent years, human intestinal epithelial stem cells have been adhered onto an extracellular matrix and cultured in the presence of a cell culture medium containing a basal medium for human cells, to which a BMP inhibitor, a mitogenic growth factor, and Wnt agonist were added, to achieve long-term culture of an organoid in which human intestinal epithelial stem cells are maintained and differentiated (for example, see PTL 1).

### Citation List

### Patent Literature

[PTL 1] Japanese Patent No. 5458112
[PTL 2] WO 2017/059449 A1
[PTL 3] EP 3 418 376 A1
[PTL 4] JP 2014 506801 A
[PTL 5] JP 2012 516685 A

### Non-Patent Literature

[NPL 1] Journal of Infection Prevention and Control, vol.24, no. 6, 2009
[NPL 2] Gastroenterology, vol. 141, 2011, pages 1762-1772.
[NPL 3] Science, vol. 353, no. 6306, 2016, pages 1387-1393.

### Summary of Invention

### Technical Problem

However, the cell culture medium of PTL 1 contains a p38 inhibitor. Therefore, in a culture method using the medium, differentiation inhibition or cell death may occur and there is room for improvement.

In order to solve the problems, an object of the present invention is to provide a method of producing human diarrhea virus in a large amount in vitro, an organoid therefor, and a production method of the organoid.

### Solution to Problem

As a result of extensive research to solve the problems, the present inventors found that infection and growth culture of human diarrhea virus are possible by using a cultured organoid obtained by improving a production method of human small intestine-derived organoid, and have completed the present invention.

That is, the present invention includes the following aspects.
[1] A production method of a 2D organoid for infection and growth culture of human diarrhea virus, comprising: a step 1 in which a human intestinal epithelial stem cell, a human intestinal epithelial cell, or a tissue containing at least any of these cells is three-dimensionally cultured on an extracellular matrix to obtain a 3D organoid; and a step 2 in which the 3D organoid obtained in the step 1 is dispersed to prepare a single cell, and the single cell is monolayer-cultured on an extracellular matrix to obtain a 2D organoid in which epithelial cells containing differentiated trophoblastic cells and goblet cells and configuring human intestinal lumen have a monolayer structure, wherein in culturing of the steps 1 and 2, a cell culture medium containing at least one selected from the group consisting of i) Wnt agonist, ii) at least one selected from the group consisting of an insulin-like growth factor 1 (IGF1), a fibroblast growth factor 2 (FGF2), an epidermal growth factor (EGF), and epiregulin (EREG), iii) a bone morphogenetic factor (bone morphogenetic protein; BMP) inhibitor, iv) a transforming growth factor-β (TGF-β) inhibitor, and v) a γ-selectase inhibitor is used, and wherein the cell culture medium contains the IGF1 and the FGF2.
[2] The production method of a 2D organoid for infection and growth culture of human diarrhea virus according to [1], in which the Wnt agonist is at least one selected from the group consisting of a Wnt protein, R-spongin, and a GSK-3β inhibitor.
[3] The production method of a 2D organoid for infection and growth culture of human diarrhea virus according to [1] or [2], in which the Wnt protein forms a complex with afamin, which is a stabilizing substance thereof.
[4] The production method of a 2D organoid for infection and growth culture of human diarrhea virus according to any one of [1] to [3], in which the Wnt protein is at least one selected from the group consisting of Wnt1, Wnt2, Wnt2b, Wnt3, Wnt3a, Wnt4, Wnt5a, Wnt5b, Wnt6, Wnt7a, Wnt7b, Wnt8a, Wnt8b, Wnt9a, Wnt9b, Wnt10a, Wnt10b, Wnt11, and Wnt16.
[5] The 2D organoid for infection and growth culture of human diarrhea virus according to any one of [1] to [4], in which the TGF-β inhibitor is at least one selected from the group consisting of A83-01, SB-431542, SB-505124, SB-525334, SD-208, LY-36494, and SJN-2511.
[6] The 2D organoid for infection and growth culture of human diarrhea virus according to [5], in which the TGF-β inhibitor is A83-01.
[7] The 2D organoid for infection and growth culture of human diarrhea virus according to any one of [1] to [6], in which the cell culture medium further contains animal-derived bile.
[8] The production method of a 2D organoid for infection and growth culture of human diarrhea virus according to any one of [2] to [7], in which the R-spongin is at least one selected from the group consisting of R-spongin 1, R-spongin 2, R-spongin 3, and R-spongin 4.
[9] The production method of 2D organoid for infection and growth culture of human diarrhea virus according to any one of [1] to [8], in which the BMP inhibitor is at least one selected from the group consisting of a chordin-like protein such as noggin, gremlin, chordin, and a chordin domain, follistatin-related protein such as follistatin and a follistatin domain, a DAN-like protein such as DAN and a DAN cysteine-knot domain, sclerostin/SOST, and α-2 macroglobulin.
[10] The production method of a 2D organoid for infection and growth culture of human diarrhea virus according to [9], in which the BMP inhibitor is noggin.
[11] The production method of a 2D organoid for infection and growth culture of human diarrhea virus according to any one of [1] to [10], in which in the steps 1 and 2, the extracellular matrix is Matrigel (registered trademark).
[12] The production method of a 2D organoid for infection and growth culture of human diarrhea virus according to any one of [1] to [11], which is peformed under serum-free.
[13] A production method of human diarrhea virus, including: a step 1 in which a human intestinal epithelial stem cell, a human intestinal epithelial cell, or a tissue containing at least any of these cells is three-dimensionally cultured on an extracellular matrix to obtain a 3D organoid; and a step 2 in which the 3D organoid obtained in the step 1 is dispersed to prepare a single cell, and the single cell is monolayer-cultured on an extracellular matrix to obtain a 2D organoid in which epithelial cells containing differentiated trophoblastic cells and goblet cells and configuring human intestinal lumen have a monolayer structure; a step 3 in which the 2D organoid is infected with human diarrhea virus and the infected 2D organoid is cultured to perform infection and growth culture of human diarrhea virus, in which in culturing of the steps 1 and 2, a cell culture medium containing at least one selected from the group consisting of i) Wnt agonist, ii) at least one selected from the group consisting of an insulin-like growth factor 1 (IGF1), a fibroblast growth factor 2 (FGF2), an epidermal growth factor (EGF), and epiregulin (EREG), iii) a bone morphogenetic factor (bone morphogenetic protein; BMP) inhibitor, iv) a transforming growth factor-β (TGF- β) inhibitor, and v) a γ-selectase inhibitor is used, and wherein the cell culture medium contains the IGF1 and the FGF2.
[14] A culture kit for a 2D organoid for infection and growth culture of human diarrhea virus, including: a cell culture medium containing at least one selected from the group consisting of i) Wnt agonist, ii) at least one selected from the group consisting of an insulin-like growth factor 1 (IGF1), a fibroblast growth factor 2 (FGF2), an epidermal growth factor (EGF), and epiregulin (EREG), iii) a bone morphogenetic factor (bone morphogenetic protein; BMP) inhibitor, iv) a transforming growth factor-β (TGF-β) inhibitor, and v) a γ-selectase inhibitor, wherein the cell culture medium contains the IGF1 and the FGF2.
[15] The culture kit for a 2D organoid for infection and growth culture of human diarrhea virus according to [14], in which the cell culture medium further contains animal-derived bile.
[16] A production kit for human diarrhea virus, including: a cell culture medium containing at least one selected from the group consisting of i) Wnt agonist, ii) at least one selected from the group consisting of an insulin-like growth factor 1 (IGF1), a fibroblast growth factor 2 (FGF2), an epidermal growth factor (EGF), and epiregulin (EREG), iii) a bone morphogenetic factor (bone morphogenetic protein; BMP) inhibitor, iv) a transforming growth factor-β (TGF-β) inhibitor, and v) a γ-selectase inhibitorwherein the Wnt agonist is a Wnt protein and R-spongin and wherein the cell culture medium contains the IGF1 and the FGF2.
[17] The production kit for human diarrhea virus according to [16], in which the Wnt protein is at least one selected from the group consisting of Wnt1, Wnt2, Wnt2b, Wnt3, Wnt3a, Wnt4, Wnt5a, Wnt5b, Wnt6, Wnt7a, Wnt7b, Wnt8a, Wnt8b, Wnt9a, Wnt9b, WntlOa, WntlOb, Wnt11, and Wnt16.
[18] The production kit for human diarrhea virus according to [16] or [17], in which the R-spongin is at least one selected from the group consisting of R-spongin 1, R-spongin 2, R-spongin 3, and R-spongin 4.
[19] The production kit for human diarrhea virus according to any one of [16] to [18], in which the BMP inhibitor is at least one selected from the group consisting of a chordin-like protein such as noggin, gremlin, chordin, and a chordin domain, follistatin-related protein such as follistatin and a follistatin domain, a DAN-like protein such as DAN and a DAN cysteine-knot domain, sclerostin/SOST, and α-2 macroglobulin.
[20] The production kit for human diarrhea virus according to any one of [16] to [19], in which the BMP inhibitor is noggin.
[21] The production kit for human diarrhea virus according to any one of [16] to [20], in which the Wnt agonist is a Wnt protein and R-spongin.
[22] The production kit for human diarrhea virus according to any one of [16] to [21], in which the TGF-β inhibitor is at least one selected from the group consisting of A83-01, SB-431542, SB-505124, SB-525334, SD-208, LY-36494, and SJN-2511.
[23] The production kit for human diarrhea virus according to any one of [16] to [22], in which the TGF-β inhibitor is A83-01.
[24] The production kit for human diarrhea virus according to any one of [16] to [23], in which the Wnt protein forms a complex with afamin, which is a stabilizing substance thereof.

### Advantageous Effects of Invention

According to the present invention, it is possible to provide a method of producing human diarrhea virus in a large amount by performing growth culture in vitro, and a production method of an organoid therefor.

### Brief Description of Drawings

FIG. 1 is an explanatory diagram of a production method of human diarrhea virus using growth culture thereof according to the present embodiment.
FIG. 2 is a graph showing results of qRT-PCR in Example 2.
FIG. 3 shows immunostained images of organoids infected with norovirus in Example 2 in an upper row, and shows schematic diagrams of those of the upper row in a lower row.
FIGS. 4(a) to 4(c) are optical micrographs of 2D organoids in Experimental Example 3.
FIGS. 5(a) and 5(b) are graphs showing results of qRT-PCR in Experimental Example 4.

### Description of Embodiments

### [Production Method of Human Diarrhea Virus]

As an embodiment, the present invention provides a production method of human diarrhea virus, including a step 1 in which a human intestinal epithelial stem cell, a human intestinal epithelial cell, or a tissue containing at least any of these cells is three-dimensionally cultured on an extracellular matrix to obtain a 3D organoid, a step 2 in which the 3D organoid obtained in the step 1 is dispersed to prepare a single cell, and the single cell is monolayer-cultured on an extracellular matrix to obtain a 2D organoid in which epithelial cells containing differentiated trophoblastic cells and goblet cells and configuring human intestinal lumen have a monolayer structure, and a step 3 in which the 2D organoid obtained in the step 2 is infected with human diarrhea virus and the infected 2D organoid is cultured to perform infection and growth culture of human diarrhea virus, in which in culturing of the steps 1, 2, and 3, a predetermined cell culture medium is used. First, the medium used for the culturing of the steps 1, 2, and 3 will be described. In the present embodiment, the medium used for the culturing of the steps 1, 2, and 3 may be all the same medium or different media.

### (Cell Culture Medium)

The medium used in the present embodiment is a cell culture medium containing at least one selected from the group consisting of i) Wnt agonist, ii) at least one selected from the group consisting of an insulin-like growth factor 1 (IGF1), a fibroblast growth factor 2 (FGF2), an epidermal growth factor (EGF), and epiregulin (EREG), iii) a bone morphogenetic factor (bone morphogenetic protein; BMP) inhibitor, iv) a transforming growth factor-β (TGF-β) inhibitor, and v) a γ-selectase inhibitor, in which the cell culture medium contains the IGF1 and the FGF2.

According to the cell culture medium of the present embodiment, it is possible to culture a human intestinal epithelial stem cell, a human intestinal epithelial cell, or a tissue containing at least any of these cells for a long period of time, without substantially containing a p38 inhibitor.

In the present specification, the expression "human intestinal epithelial cell" includes a differentiated human intestinal epithelial cell and human intestinal epithelial stem cell obtained from a human intestinal epithelial tissue. The expression "human intestinal epithelial stem cell" means a cell having a long-term self-reproduction function and differentiation ability into a human intestinal epithelial differentiated cell, and means a stem cell derived from a human intestinal epithelial tissue.

In the present specification, the expression "organoid" means a three-dimensional cellular organism which is self-organized by densely accumulating cells in a controlled space.

In the present specification, the expression "without substantially containing" means that a specific component is not contained at all or contained only at a concentration at which a function of the specific component is not exerted.

Accordingly, the expression "without substantially containing a p38 inhibitor" means that the p38 inhibitor is not contained or contained only at a concentration at which differentiation inhibition and cell death by the p38 inhibitor do not occur.

The cell culture medium according to the present embodiment contains the IGF 1 and the FGF2. The cell culture medium according to the present embodiment may further contain at least one selected from the group consisting of EGF and epiregulin.

When containing the factors, even without substantially containing the EGF and the p38 inhibitor, it is possible to form an organoid with high efficiency from the human intestinal epithelial stem cell, the human intestinal epithelial cell, or the tissue containing at least one of these cells.

### (Basal Medium for Cell Culturing)

The cell culture medium according to the present embodiment includes all serum-free basal medium for cell culturing. The cell culture medium according to the present embodiment is preferably used for a human cell.

Examples of the serum-free basal medium for cell culturing include a specified synthetic medium which is buffered with a carbonate type buffer solution to pH 7.2 or higher and pH 7.6 or lower. More specifically, examples thereof include an advanced Dulbecco's Modified Eagle Medium: Nutrient Mixture F-12 (DMEM/F12) which is supplemented with glutamine, insulin, B27 supplement (Thermo Fisher), N-Acetyl-L-cystein (Wako Pure Chemical Industries), penicillin or streptomycin, and transferrin.

In addition, examples thereof also include a Roswell Park Memorial Institute 1640 medium (PRMI 1640 medium), DMEM/F12, and an advanced RPMI medium, instead of the advanced Dulbecco's Modified Eagle Medium: Nutrient Mixture F-12.

The cell culture medium according to the present embodiment does not substantially contain a component such as fetal bovine serum (FBS) or fetal calf serum. In addition, 5% serum may be contained in the cell culture medium.

### (Wnt Agonist)

In the present specification, the "Wnt agonist" means a drug which activates transcription mediated by a T-cell factor (hereinafter, also referred to as a TCF)/lymphoid enhancer factor (hereinafter, also referred to as an LEF). Accordingly, the Wnt agonist is not limited to a Wnt family protein, and includes a Wnt agonist that is bonded to a Frizzled receptor family member to be activated, an intracellular β-catenin degradation inhibitor, and an activator of the TCF/LEF. The Wnt agonist is preferably at least one selected from the group consisting of a Wnt protein, R-spongin, and a GSK-3β inhibitor, and more preferably the Wnt protein and the R-spongin.

The Wnt agonist stimulates Wnt activity in a cell by at least 10%, preferably at least 20%, more preferably at least 30%, still more preferably at least 50%, particularly preferably at least 90%, and most preferably 100%, compared to a level of Wnt activity in the absence of the Wnt agonist. The Wnt activity can be examined by measuring the transcriptional activity of the Wnt, using a method known to those skilled in the art, for example, by pTOPFLASH and pFOPFLASH Tcf luciferase reporter constructs (reference: Korinek et al., 1997. Science 275: 1784-1787).

In culturing of the human intestinal epithelial stem cell, the human intestinal epithelial cell, or the tissue containing at least any of these cells, it is preferable to contain the Wnt agonist. As the Wnt agonist to be contained in the cell culture medium according to the present embodiment, it is more preferable to contain a complex of a Wnt protein and afamin, and still more preferable to contain both the complex of the Wnt protein and the afamin and R-spongin (R-spondin). In the cell culture medium according to the present embodiment, the human intestinal epithelial stem cell or the human intestinal epithelial cell can form an organoid more efficiently by containing the complex of the Wnt protein and the afamin and the R-spongin.

### (Wnt Protein)

An origin of the Wnt protein, which is one kind of the Wnt agonist, is not particularly limited, and Wnt proteins derived from various organisms can be used. Among these, a Wnt protein derived from a mammal is preferable. Examples of the mammal include human, mouse, rat, cow, pig, and rabbit. Examples of the Wnt protein of the mammal include Wnt1, Wnt2, Wnt2b, Wnt3, Wnt3a, Wnt4, Wnt5a, Wnt5b, Wnt6, Wnt7a, Wnt7b, Wnt8a, Wnt8b, Wnt9a, Wnt9b, WntlOa, WntlOb, Wnt11, and Wnt16. In the cell culture medium according to the present embodiment, plural kinds of the Wnt proteins may be used in combination.

Examples of a method of producing the Wnt protein include a production method using a Wnt protein-expressing cell. In the Wnt protein-expressing cell, an origin (such as biological species or a culture form) of the cell is not particularly limited, as long as it is a cell stably expressing the Wnt protein. The Wnt protein-expressing cell may also be a cell transiently expressing the Wnt protein. Examples of the Wnt protein-expressing cell include an L cell (ATCC CRL-2647) stably expressing mouse Wnt 3a and an L cell (ATCC CRL-2814) stably expressing mouse Wnt 5a. In addition, The Wnt protein-expressing cells can be produced using known genetic recombination techniques. That is, a Wnt protein-expressing cell can be prepared by inserting a DNA encoding a desired Wnt protein into a known expression vector and introducing the obtained expression vector into an appropriate host cell. A nucleotide sequence of a gene encoding a desired Wnt protein can be obtained from a known database such as GenBank, for example.

The Wnt protein expressed by the Wnt protein-expressing cell may be a fragment of the Wnt protein or may include an amino acid sequence other than the amino acid sequence of the Wnt protein as long as it has Wnt activity. The amino acid sequence other than the amino acid sequence of the Wnt protein is not particularly limited, and examples thereof include an amino acid sequence of an affinity tag. In addition, the amino acid sequence of the Wnt protein does not necessarily need to be completely identical with the amino acid sequence that can be obtained from the known database such as GenBank. The amino acid sequence of the Wnt protein may be substantially the same amino acid sequence as the amino acid sequence that can be obtained from the known database, as long as it has Wnt activity.

Examples of substantially the same amino acid sequence as the amino acid sequence of the Wnt protein that can be obtained from the known database such as GenBank include an amino acid sequence, in which one to several amino acids are deleted, substituted, or added, in the amino acid sequence that can be obtained from the known database.

The expression "amino acid sequence, in which one to several amino acids are deleted, substituted, or added," means that an amino acid is deleted, substituted, or added by the number (preferably 10 or less, more preferably 7 or less, and still more preferably 6 or less) by which deletion, substitution, or addition can be performed by a known mutant peptide preparation method such as site-directed mutagenesis.

Examples of substantially the same amino acid sequence include an amino acid sequence in which an identity with the amino acid sequence that can be obtainable from the known database is at least 80% or more, preferably at least 85% or more, more preferably at least 90% or more, still more preferably at least 92% or more, particularly preferably at least 95% or more, and most preferably at least 99% or more.

The activity of the Wnt protein can be confirmed by, for example, TCF reporter assay. In general, the TCF reporter assay is a method in which a luciferase gene having a binding sequence of a T-cell factor (TCF), which is a transcription factor specifically activated when a Wnt signal enters a cell, is introduced and an intensity of the Wnt protein activity is conveniently evaluated by luminescence of luciferase (Reference: Molenaar et al., Cell, 86, 391, 1996). Examples of a method other than the TCF reporter assay include a method in which the amount of β-catenin is quantitatively evaluated by western blotting using the fact that intracellular β-catenin stabilizes when the Wnt signal enters the cell (Reference: Shibamoto et al., Gene to cells, 3, 659, 1998). In addition, in a Wnt protein such as Wnt 5a, which gives a signal to a cell via a non-canonical pathway, a method in which the activity of the Wnt protein is evaluated by evaluating phosphorylation of DvL2, which is an intracellular adapter protein, can be used (Reference: Kikuchi et al., EMBO J., 29, 3470, 2010).

### (R-Spongin (R-Spondin))

Examples of the R-spongin, which is a kind of the Wnt agonist, include an R-spongin family including R-spongin 1, R-spongin 2, R-spongin 3, and R-spongin 4.

The R-spongin family is a secreted protein and is known to be involved in activation and regulation of the Wnt signal transduction pathway. In the cell culture medium according to the present embodiment, plural kinds of the R-spongins may be used in combination.

The R-spongin may be a fragment of the R-spongin or may include an amino acid sequence other than the amino acid sequence of the R-spongin as long as it has R-spongin activity.

### (Content)

A concentration of the Wnt protein contained in the cell culture medium according to the present embodiment is preferably 50 ng/mL or more, more preferably 100 ng/mL or more and 10 µg/mL or less, still more preferably 200 ng/mL or more and 1 µg/mL or less, and particularly preferably 300 ng/mL or more and 1 µg/mL or less. During culturing of the human intestinal epithelial stem cell, it is preferable to add the Wnt agonist to the culture medium every 2 days and it is preferable to replace the cell culture medium with a new one every 4 days.

### (GSK-3β inhibitor)

A known GSK-3β inhibitor includes CHIR-99021 and CHIR-98014 (Sigma-Aldrich), Lithium (Sigma), Kenpaullone (Biomol International, Leost, M. et al. (2000) Eur J Biochem 267, 5983-5994), 6-bromoindirubin-30-acetoxime (Meyer, L et al. (2003), Chem. Biol. 10, 1255-1266), SB 216763 and SB415286 (Sigma-Aldrich), and FRAT family member, which prevents GSK-3 and axin from interacting with each other, and FRAT-derived peptide. An outline thereof is shown in Meijer et al. (2004) Trends in Pharmacological Sciences 25, 471-480, which is incorporated in the present specification by reference. A method and an assay for determining a level of GSK-3 β inhibition are known to those skilled in the art, and examples thereof include a method and an assay described in Liao et al. (2004), Endocrinology, 145(6), 2941-2949.

### (Afamin)

In the present specification, "afamin" means a glycoprotein belonging to an albumin family and is known to exist in blood or body fluids. A serum usually added to a medium used for cell culturing contains afamin derived from the animal from which the serum was collected. Since the serum contains impurities and the like other than afamin, in the cell culture medium according to the present embodiment, it is preferable to use afamin alone without using a serum.

An origin of the afamin contained in the cell culture medium according to the present embodiment is not particularly limited, and afamin derived from various organisms can be used. Among these, afamin derived from a mammal is preferable. Examples of the mammal include the same as in the above-described [Wnt Protein]. The main amino acid sequence of the afamin of the mammal and a nucleotide sequence of a gene encoding the same can be obtained from a known database such as GenBank, for example. For example, in GenBank, an amino acid sequence of human afamin is registered with the accession number of AAA21612, and a nucleotide sequence of the gene encoding the same is registered with the accession number of L32140. An amino acid sequence of bovine afamin is registered with the accession number of DAA28569, and a nucleotide sequence of the gene encoding the same is registered with the accession number of GJ060968.

The afamin contained in the cell culture medium according to the present embodiment may be obtained by purifying a natural afamin contained in serum or the like by a known method or may be a recombinant afamin. The recombinant afamin can be produced by appropriately using known genetic recombination techniques.

As a production method of the recombinant afamin, for example, the recombinant afamin can be produced by inserting a DNA encoding afamin into a known expression vector, introducing the obtained expression vector into an appropriate host cell to express recombinant afamin, and purifying the same by using a known purification method. The recombinant afamin may also be afamin to which an affinity tag is added. The affinity tag to be added is not particularly limited, and a known affinity tag can be appropriately selected and used. The affinity tag is preferably an affinity tag recognized by a specific antibody, and examples thereof include a FLAG tab, an MYC tag, an HA tag, and V5 tag.

In the above-described Wnt protein, since a specific serine residue is modified with a fatty acid (palmitoleic acid), the Wnt protein has strong hydrophobicity. Therefore, it is widely known that since the Wnt protein tends to aggregate or denature in an aqueous solution, purification and preservation thereof are very difficult.

On the other hand, the modification of the specific serine residue with the fatty acid has been reported to be essential for physiological activity of the Wnt protein and involved in bonding to Frizzled receptor family member.

In addition, there is also a finding that in an aqueous solution, the Wnt protein binds to afamin one to one to form a complex and is solubilized while maintaining high physiological activity (Active and water-soluble form of lipidated Wnt protein is maintained by A serum glycoprotein afamin/a-albumin.Mihara E, Hirai H, Yamamoto H, Tamura-Kawakami K, Matano M, Kikuchi A, Sato T, Takagi J. Elife. 2016 Feb 23; 5).

On the basis of the finding, a Wnt protein-afamin complex may be produced by a method of culturing cells expressing both the Wnt protein and the afamin, or a Wnt protein-afamin complex may be produced by a method of coculturing the Wnt protein-expressing cell and an afamin expressing cell. The activity of the Wnt protein in the Wnt protein-afamin complex can be evaluated by using the same method as in the above-described [Wnt Protein].

A concentration of the afamin contained in the cell culture medium according to the present embodiment is not particularly limited, but is preferably 50 ng/mL or more and 10 µg/mL or less, more preferably 100 ng/mL or more and 1 µg/mL or less, and still more preferably 300 µg/mL or more and 1 µg/mL or less.

### (Insulin-like Growth Factor 1 (IGF1))

In general, the "insulin-like growth factor 1 (IGF1)" is also called somatomedin C as a synonym, and is a factor mainly secreted by stimulation by growth hormone (GH) in the liver. It is known that most cells of the human body (in particular, cells such as muscle, bone, liver, kidney, nerve, skin, and lung) are affected by the IGF1. In addition to an insulin-like effect, the IGF1 has a function of regulating cell growth (in particular, nerve cells) and development thereof, and cellular DNA synthesis.

A concentration of the IGF1 contained in the cell culture medium according to the present embodiment is not particularly limited, but is preferably 5 ng/mL or more and 1 µg/mL or less, more preferably 10 ng/mL or more and 1 µg/mL or less, and still more preferably 50 ng/mL or more and 500 ng/mL or less.

When the concentration of the IGF1 contained in the cell culture medium according to the present embodiment is within the above ranges, it is possible to culture a human intestinal epithelial stem cell, a human intestinal epithelial cell, or a tissue containing at least any of these cells for a long period of time, without substantially containing the p38 inhibitor.

In addition, it is possible to form an organoid with high efficiency from the human intestinal epithelial stem cell, the human intestinal epithelial cell, or the tissue containing at least one of these cells.

In addition, during culturing of the human intestinal epithelial stem cell, it is preferable to add the IGF1 to the culture medium every 2 days and it is preferable to replace the culture medium with a new one every 4 days.

### (Fibroblast Growth Factor 2 (FGF2))

In general, the "fibroblast growth factor 2 (FGF2)" is a basic fibroblast growth factor which binds to a fibroblast growth factor receptor (FGFR) and promotes growth of vascular endothelial cells and organizes this into a tubular structure, that is, has a function of promoting angiogenesis. In addition, it is known that human FGF2 has two isoforms of a low-molecular-weight form (LWL) and a high-molecular-weight form (HWL). The LWL is mainly present a cytoplasm and acts autocrine, whereas the HWL is in the nucleus and shows activity in an intracrine mechanism acting intracellularly.

A concentration of the FGF2 contained in the cell culture medium according to the present embodiment is not particularly limited, but is preferably 5 ng/mL or more and 1 µg/mL or less, more preferably 10 ng/mL or more and 1 µg/mL or less, and still more preferably 50 ng/mL or more and 500 ng/mL or less.

When the concentration of the FGF2 contained in the cell culture medium according to the present embodiment is within the above ranges, it is possible to culture a human intestinal epithelial stem cell, a human intestinal epithelial cell, or a tissue containing at least any of these cells for a long period of time, without substantially containing the p38 inhibitor.

In addition, it is possible to form an organoid with high efficiency from the human intestinal epithelial stem cell, the human intestinal epithelial cell, or the tissue containing at least one of these cells.

In addition, during culturing of the human intestinal epithelial stem cell, it is preferable to add the FGF2 to the culture medium every 2 days and it is preferable to replace the culture medium with a new one every 4 days.

### (Epiregulin (EREG))

In general, "epiregulin (EREG)" is an EGF-like growth factor that specifically binds to ErbB 1 and ErbB4 among tyrosine kinase (ErbB) family receptors (ErbB 1 to ErbB4). It is known to stimulate the growth of a keratinocyte, a hepatocyte, a fibroblast, and a vascular endothelial cell. In addition, EREG is mainly expressed in cancer tumors of the bladder, lung, kidney, and large intestine, placenta, and peripheral blood leukocytes.

A concentration of EREG contained in the cell culture medium according to the present embodiment is not particularly limited, but is preferably 5 ng/mL or more and 1 µg/mL or less, more preferably 10 ng/mL or more and 1 µg/mL or less, and still more preferably 50 ng/mL or more and 500 ng/mL or less.

When the concentration of EREG contained in the cell culture medium according to the present embodiment is within the above ranges, it is possible to culture a human intestinal epithelial stem cell, a human intestinal epithelial cell, or a tissue containing at least any of these cells for a long period of time, without substantially containing the p38 inhibitor.

In addition, it is possible to form an organoid with high efficiency from the human intestinal epithelial stem cell, the human intestinal epithelial cell, or the tissue containing at least one of these cells.

In addition, during culturing of the human intestinal epithelial stem cell, it is preferable to add EREG to the culture medium every 2 days and it is preferable to replace the culture medium with a new one every 4 days.

### (BMP inhibitor)

The bone morphogenetic factor (bone morphogenetic protein; BMP) binds to a receptor complex including two different kinds of receptor serine/threonine kinases and Type I and Type II receptors, as dimeric ligands. The Type II receptor phosphorylates the Type I receptor. As a result, the receptor kinase is activated. The Type I receptor subsequently phosphorylates a specific receptor substrate (SMAD). As a result, transcriptional activity is induced by a signal transduction pathway. In general, the BMP inhibitor is a drug which prevents or inhibits a BMP molecule from binding to a BMP receptor and binds to the BMP molecule to form a complex that neutralizes BMP activity. In addition, the BMP inhibitor is, for example, a drug which binds to the BMP receptor, prevents or inhibits the BMP molecule from binding to the receptor, and acts as an inhibitor or inverse agonist.

The BMP inhibitor has inhibitory activity of preferably 50% or more, more preferably 70% or more, still more preferably 80% or more, and particularly preferably 90 or more, compared to a level of BMP activity in the absence of an inhibitor thereof. The BMP inhibitory activity can be evaluated by measuring a transcriptional activity of BMP, by using a method known to those skilled in the art (Reference: Zilberberg et al., BMC Cell Biol, 8: 41, 2007).

As the BMP inhibitor contained in the cell culture medium according to the present embodiment, a natural BMP-binding protein is preferable. Examples thereof include a chordin-like protein such as noggin, gremlin, chordin, and a chordin domain; follistatin-related protein such as follistatin and a follistatin domain; a DAN-like protein such as DAN and a DAN cysteine-knot domain; sclerostin/SOST, decorin, and α-2 macroglobulin.

As a BMP inhibitor contained in the cell culture medium according to the present embodiment, among them, the chordin-like protein or the DAN-like protein is preferable, and the chordin-like protein is more preferable. As the chordin-like protein, the noggin is preferable. The chordin-like protein or the DAN-like protein is a diffusible protein, and can bind to a BMP molecule with various affinities to inhibit approach of the BMP molecule to a signal transduction receptor. In the case of culturing an epithelial stem cell, it is possible to prevent the stem cell from being lost, by adding these BMP inhibitors to the cell culture medium.

A concentration of the BMP inhibitor contained in the cell culture medium according to the present embodiment is preferably 10 ng/mL or more and 100 ng/mL or less, more preferably 20 ng/mL or more and 100 ng/mL or less, and still more preferably 50 ng/mL or more and 100 ng/mL or less. During culturing of a stem cell, it is preferable to add the BMP inhibitor to the culture medium every 2 days and it is preferable to replace the culture medium with a new one every 4 days.

### (TGF-β inhibitor)

The transforming growth factor-β (TGF-β) is one kind of growth factor and is produced in almost all cells such as kidney, bone marrow, and platelets. There are five subtypes (β1 to β5) in TGF-β. In addition, it is also known that TGF-β promotes synthesis and growth of a connective tissue such as collagen and acts on growth of the epithelial cell or on an osteoclast in an inhibitory manner. In general, the TGF-β inhibitor is a drug which prevents or inhibits a TGF-β from binding to a TGF-β receptor and binds to the TGF-β to form a complex that neutralizes TGF-β activity. In addition, the TGF-β inhibitor is, for example, a drug which binds to the TGF-β receptor, prevents or inhibits the TGF-β from binding to the receptor, and acts as inhibitor or inverse agonist.

The TGF-β inhibitor has inhibitory activity of preferably 50% or more, more preferably 70% or more, still more preferably 80% or more, and particularly preferably 90 or more, compared to a level of TGF-β activity in the absence of an inhibitor thereof. The TGF-β inhibitory activity can be evaluated by a method known to those skilled in the art. Examples of the evaluation system include a cell assay in which cells are stably transfected using a human PAI-1 promoter that drives a luciferase reporter gene or a reporter construct that contains a Smad binding site (Reference: De Gouville et al., Br J Pharmacol, 145 (2): 166-177, 2005).

Examples of the TGF-β inhibitor contained in the cell culture medium according to the present embodiment include A83-01 (3-(6-methylpyridin-2-yl)-1-phenylthiocarbamoyl-4-quinolin-4-ylpyrazole), ALK 5 Inhibitor I (3-(pyridin-2-yl)-4-(4-quinonyl)-1H-pyrazole), LDN 193189 (4-(6-(4-(piperazin-1-yl)phenyl)pyrazolo [1,5-a]pyrimidin-3-yl)quinoline), SB 431542 (4-[4-(1,3-benzodioxol-5-yl)-5-pyridin-2-yl-1H-imidazol-2-yl]benzamide), SB-505124 (2-(5-benzo[1,3]dioxol-5-yl-2-tert-butyl-3H-imidazol-4-yl)-6-methylpyridine hydrochloride hydrate), SD-208 (2-(5-chloro-2-fluorophenyl)pteridin-4-yl)pyridin-4-yl-amine), SB-525334 (6-[2-(1,1-dimethylethyl)-5-(6-methyl-2-pyridinyl)-1H-imidazol-4-yl]quinoxaline), LY-364947 (4-[3-(2-pyridinyl)-1H-pyrazol-4-yl]-quinoline), LY 2157299 (4-[2-(6-methyl-pyridin-2-yl)-5,6-dihydro-4H-pyrrolo [1,2-b]pyrazol-3-yl]-quinoline-6-carboxamide, TGF-β RI Kinase Inhibitor II 616452 (2-(3-(6-methylpyridin-2-yl)-1H-pyrazol-4-yl)-1,5-naphthyridine), TGF-β RI Kinase Inhibitor III 616453 (2-(5-benzo[1,3]dioxol-4-yl-2-tert-butyl-1H-imidazol-4-yl)-6-methylpyridine, HCl), TGF-β RI Kinase Inhibitor IX 616463 (4-((4-((2,6-dimethylpyridin-3-yl)oxy)pyridin-2-yl)amino)benzenesulfonamide), TGF-β RI Kinase Inhibitor VII 616458 (1-(2-((6,7-dimethoxy-4-quinolyl)oxy)-(4,5-dimethylphenyl)-1-ethanone), TGF-β RI Kinase Inhibitor VIII 616459 (6-(2-tert-butyl-5-(6-methyl-pyridin-2-yl)-1H-imidazol-4-yl)-quinoxaline), AP 12009 (TGF-β2 antisense compound "Trabedersen"), Belagenpumatucel-L (TGF-β2 antisense gene modified allogeneic tumor cell vaccine), CAT-152 (Glaucoma-lerdelimumab (anti-TGF-β-2 monoclonal antibody)), CAT-192 (Metelimumab (human IgG4 monoclonal antibody neutralizing TGFβ1), and GC-1008 (anti-TGF-β monoclonal antibody). As the TGF-β inhibitor contained in the cell culture medium according to the present embodiment, among them, A83-01 is preferable.

A concentration of the TGF-β inhibitor contained in the cell culture medium according to the present embodiment is preferably 100 nM or more and 10 µM or less, more preferably 500 nM or more and 5 µM or less, and still more preferably 500 nM or more and 2 µM or less. During culturing of a stem cell, it is preferable to add the TGF-β inhibitor to the culture medium every 2 days and it is preferable to replace the culture medium with a new one every 4 days.

### (γ-Selectase Inhibitor)

The γ-selectase inhibitor has inhibitory activity of preferably 50% or more, more preferably 70% or more, still more preferably 80% or more, and particularly preferably 90 or more, compared to a level of γ-selectase activity in the absence of an inhibitor thereof. The γ-selectase inhibitory activity can be evaluated by a method known to those skilled in the art.

Examples of the γ-selectase inhibitor contained in the cell culture medium according to the present embodiment include BMS 299897, DAPT, DBZ, JLK6, L-685, 458, and LY411575. As the γ-selectase inhibitor contained in the cell culture medium according to the present embodiment, among them, LY 411575 is preferable.

A concentration of the γ-selectase inhibitor contained in the cell culture medium according to the present embodiment is preferably 1 nM or more and 10 µM or less and still more preferably 100 nM or more and 1 µM or less.

From the viewpoint of increasing the production amount of the human diarrhea virus per cell, it is preferable to use the γ-selectase inhibitor in monolayer culture of the step 2. During culturing of a stem cell, it is preferable to add the γ-selectase inhibitor to the culture medium every 2 days and it is preferable to add the γ-selectase inhibitor to the culture medium for 4 days.

### (Mitogenic Growth Factor)

Examples of the mitogenic growth factor which may be contained in the cell culture medium according to the present embodiment include an epidermal growth factor (EGF). Also disclosed herein are other members of the growth factor family: a brain-derived neurotrophic factor (BDNF), and a keratinocyte growth factor (KGF). Plural kinds of these mitogenic growth factors may be used in combination.

The EGF is a potent mitogenic factor for various cultured ectodermal cells and mesodermal cells, and has a remarkable effect on the differentiation of specific cells of some fibroblasts. An EGF precursor exists as a membrane-bound molecule that is cleaved by proteolysis to produce a 53-amino acid peptide hormone that stimulates the cell.

EGF may be included in the cell culture medium. A concentration of the EGF contained in the cell culture medium according to the present embodiment is preferably 5 ng/mL or more and 500 ng/mL or less, more preferably 10 ng/mL or more and 400 ng/mL or less, and still more preferably 50 ng/mL or more and 200 ng/mL or less.

In addition, in the case where the cell culture medium further contains KGF, the content is preferably the same as that of KGF. In the case where a plurality of KGFs such as KGF1 and KGF2 (also known as FGF7 and FGF10) are used, a total content of the KGF is preferably within the above ranges. During culturing of a stem cell, it is preferable to add the mitogenic growth factor to the culture medium every 2 days and it is preferable to replace the culture medium with a new one every 4 days.

### (Other Components)

The cell culture medium according to the present embodiment may further contain a Rock (Rho-kinase) inhibitor. Examples of the Rock inhibitor include Y-27632 ((R)-(+)-trans-4-(1-aminoethyl)-N-(4-pyridyl)cyclohexane carboxamide dihydrochloride monohydrate), Fasudil (HA 1077) (5-(1,4-diazepan-1-yl sulfonyl)isoquinoline), and H-1152 ((S)-(+)-2-methyl-1-[(4-methyl-5-isoquinolinyl)sulfonyl]-hexahydro-1H-1,4-diazepine dihydrochloride). In the case where Y-27632 is used as the Rock inhibitor, it is preferable to add the Y-27632 in the first two days of culturing the stem cell dispersed in a single cell. It is preferable that the Y-27632 contained in the cell culture medium according to the present embodiment be approximately 10 µM.

Gastrin (or a suitable substitute such as Leu 15-gastrin) may be further added to the cell culture medium according to the present embodiment. A concentration of the gastrin (or a suitable substitute) contained in the cell culture medium according to the present embodiment may be 1 ng/mL or more and 10 µg/mL, for example, may be 1 ng/mL or more and 1 µg/mL or less, and for example, may also be 5 ng/mL or more and 100 ng/mL or less.

The cell culture medium according to the present embodiment may further contain at least one kind of amino acid. Examples of the amino acid contained in the cell culture medium according to the present embodiment include L-alanine, L-arginine, L-asparagine, L-aspartic acid, L-cysteine, L-cystine, L-glutamic acid, L-glutamine, L-glycine, L-histidine, L-isoleucine, L-leucine, L-lysine, L-methionine, L-phenylalanine, L-proline, L-serine, L-threonine, L-tryptophan, L-tyrosine, and L-valine, and a combination thereof. In general, a concentration of the L-glutamine contained in the cell culture medium is 0.05 g/L or more and 1 g/L or less (usually 0.1 g/L or more and 0.75 g/L or less). Other amino acids contained in the cell culture medium are 0.001 g/L or more and 1 g/L (usually, 0.01 g/L or more and 0.15 g/L or less). The amino acid may also be derived by synthesis.

The cell culture medium according to the present embodiment may further contain at least one kind of vitamin. Examples of the vitamin contained in the cell culture medium according to the present embodiment include thiamine (vitamin B1), riboflavin (vitamin B2), niacin (vitamin B3), D-calcium pantothenate (vitamin B5), pyridoxal/pyridoxamine/pyridoxine (vitamin B6), folic acid (vitamin B9), cyanocobalamin (vitamin B12), ascorbic acid (vitamin C), calciferol (vitamin D2), DL-α tocopherol (vitamin E), biotin (vitamin H), and menadione (vitamin K).

The cell culture medium according to the present embodiment may further contain at least one kind of an inorganic salt. The inorganic salt contained in the cell culture medium according to the present embodiment is to assist maintenance of osmotic equilibrium of a cell and assist regulation of membrane potential. Specific examples of the inorganic salt include salts of calcium, copper, iron, magnesium, potassium, sodium, and zinc. Salts are usually used in forms of chloride, phosphate, sulfate, nitrate, and bicarbonate. Further, specific examples of the salts include CaCl₂, CuSO₄-5H₂O, Fe(NO₃)-9H₂O, FeSO₄-7H₂O, MgCl, MgSO₄, KCL, NaHCO₃, NaCl, Na₂HPO₄, Na₂HPO₄-H₂O, and ZnSO₄-7H₂O.

The cell culture medium according to the present embodiment may further contain at least one kind of a saccharide as a carbon energy source. Examples of the saccharide contained in the cell culture medium according to the present embodiment include glucose, galactose, maltose, and fructose. Among them, as the saccharide, glucose is preferable and D-glucose (dextrose) is particularly preferable. It is preferable that a concentration of the saccharide contained in the cell culture medium according to the present embodiment be 1 g/L or more and 10 g/L.

The cell culture medium according to the present embodiment may further contain at least one kind of a microelement. Examples of the microelements contained in the cell culture medium according to the present embodiment include barium, bromium, cobalt, iodine, manganese, chromium, copper, nickel, selenium, vanadium, titanium, germanium, molybdenum, silicon, iron, fluorine, silver, rubidium, tin, zirconium, cadmium, zinc, and aluminum or ions thereof.

The cell culture medium according to the present embodiment may further contain at least one kind of an additional drug. Examples of the drug include a nutrient or a growth factor reported to improve stem cell culturing, such as cholesterol/transferrin/albumin/insulin/progesterone, putrescine, and selenite/other factors.

The cell culture medium according to the present embodiment may further contain animal-derived bile. As described later in Examples, it is possible to remarkably promote growth of the human diarrhea virus, by adding the animal-derived bile to the cell culture medium. Examples of the animal-derived bile include bovine bile extract and swine bile extract. These bile extracts may also be commercially available bile extracts.

### (Step 1)

The step 1 is a step in which the human intestinal epithelial stem cell, the human intestinal epithelial cell, or the tissue containing at least any of these cells is three-dimensionally cultured on the extracellular matrix to obtain a 3D organoid.

In the step 1, it is preferable to include an extracellular matrix preparation step in which an extracellular matrix is prepared, an adhesion step in which a human intestinal epithelial stem cell, a human intestinal epithelial cell, or a tissue containing at least any of these cells in the extracellular matrix is adhered onto an extracellular matrix, an organoid-forming step in which the cell culture medium is added after the adhesion step, and the human intestinal epithelial stem cell, the human intestinal epithelial cell, or the tissue containing at least any of these cells is cultured to form an organoid. Preferable steps included in the step 1 will be described in detail below.

### «Extracellular Matrix Preparation Step»

In general, the "extracellular matrix (ECM)" means a supramolecular structure existing outside a cell in an organism. The ECM serves as a scaffolding for growth of an epithelial stem cell, an epithelial tumor cell, or tissues containing them.

The ECM contains various polysaccharides, water, elastin, and glycoprotein. Examples of the glycoprotein include collagen, entactin (nidogen), fibronectin, and laminin.

Examples of a preparation method of the ECM include a method using a connective tissue cell. More specifically, ECM-producing cells such as fibroblasts are cultured. Thereafter, these cells are extracted, and an epithelial stem cell, an epithelial cell, an epithelial tumor cell, or a tissue containing them is added thereto. Accordingly, the ECM can be used as a scaffolding.

Examples of the ECM-producing cells include chondrocytes which mainly produce collagen and proteoglycan, fibroblasts which mainly produce Type IV collagen, laminin, interstitial procollagen, and fibronectin, and colonic myofibroblasts which produce collagen (Type I, Type III, and Type V), chondroitin sulfate proteoglycan, hyaluronic acid, fibronectin, and tenascin-C. Alternatively, commercially available ECM may be used. Examples of commercially available ECM include a basement membrane preparation (for example, Matrigel (registered trademark) (manufactured by BD Biosciences)) derived from extracellular matrix proteins (manufactured by Invitrogen) and Engelbreth-Holm-Swarm (EHS) mouse sarcoma cells. Synthetic ECM such as ProNectin (Sigma Z378666) may be used. In addition, a mixture of natural ECM and synthetic ECM may also be used.

In the case where the ECM is used for culturing a stem cell, it is possible to enhance long-term survival of the stem cell and continued presence of undifferentiated stem cells. In the absence of the ECM, the stem cell cultures cannot be cultured over a long period of time and the continued presence of undifferentiated stem cells is not observed. Further, when the ECM is present, it is possible to culture an organoid that cannot be cultured in the absence of the ECM.

The ECM is usually sunk to the bottom of a dish where cells are suspended. For example, when the ECM coagulates at 37°C, the above-described cell culture medium may be added and used by diffusing into the ECM. The cells in the medium can be fixed to the ECM by interacting with a surface structure of the ECM, for example, by interacting with an integrin.

The ECM may be used in a state where a culture vessel or the like is coated with the ECM. In the case where fibronectin is used as the ECM, a ratio by which the fibronectin is coated on the culture vessel is preferably 1 µg/cm² or more and 250 µg/cm² or less, more preferably 1 µg/cm² or more and 150 µg/cm² or less, and still more preferably 8 µg/cm² or more and 125 µg/cm² or less.

As the extracellular matrix, Matrigel (registered trademark) is preferably used. When using Matrigel (registered trademark) as the extracellular matrix as described later in the examples, adhesion of the 2D organoid to a culture substrate tends to be favorable.

### <<Adhesion Step>>

Subsequently, a human intestinal epithelial stem cell, a human intestinal epithelial cell, or a tissue containing at least any one of these cells is prepared.

Examples of a method of isolating the human intestinal epithelial cell from a human intestinal epithelial tissue include a method known in the technical field. For example, it is possible to isolate crypts by incubating a chelating agent and an isolated tissue. After washing the tissue, a human intestinal epithelial cell layer is peeled off from a submucosal layer in a glass slide, and cut into small pieces. Thereafter, it is incubated in a liquid containing trypsin or preferably at least any one of EDTA and EGTA, and undigested tissue fragments and crypt-derived single cells are separated, for example, by using at least any one of filtration and centrifugation. Instead of the trypsin, at least any one of other proteolytic enzymes such as collagenase and dispase I may be used.

Examples of a method of isolating a stem cell from a human intestinal epithelial tissue include a method known in the technical field. The stem cell expresses at least any one of Lgr5 and Lgr6 on a surface thereof (Lgr5 and Lgr6 belong to a large G protein-coupled receptor (GPCR) superfamily). Examples of the isolation method include a method in which a cell suspension is prepared from a human intestinal epithelial tissue, the cell suspension is brought into contact with a chemical substance which binds to at least any one of the Lgr5 and the Lgr6, and the chemical substance which binds to at least any one of the Lgr5 and the Lgr6 is separated to isolate stem cells from the binding compound.

Examples of the chemical substance which binds to at least any one of Lgr5 and Lgr6 include an antibody. More specific examples thereof include a monoclonal antibody (such as a monoclonal antibody including mouse and rat monoclonal antibodies) which specifically recognizes at least any one of Lgr5 and Lgr6 and binds thereto. It is possible to isolate stem cells expressing at least any one of Lgr5 and Lgr6 by using such antibodies, for example, utilizing magnetic beads or through a fluorescence-activated cell sorter.

A human intestinal epithelial stem cell, a human intestinal epithelial cell, a human intestinal epithelial tumor cell, or a tissue containing at least any one of these cells which was isolated by the above method is embedded in the cell matrix obtained in the preparation step, seeded on a plate, and allowed to stand. The seeded cells can adhere to the ECM by interacting with a surface structure of the ECM, for example, by interacting with an integrin.

### <<Organoid-Forming Step>>

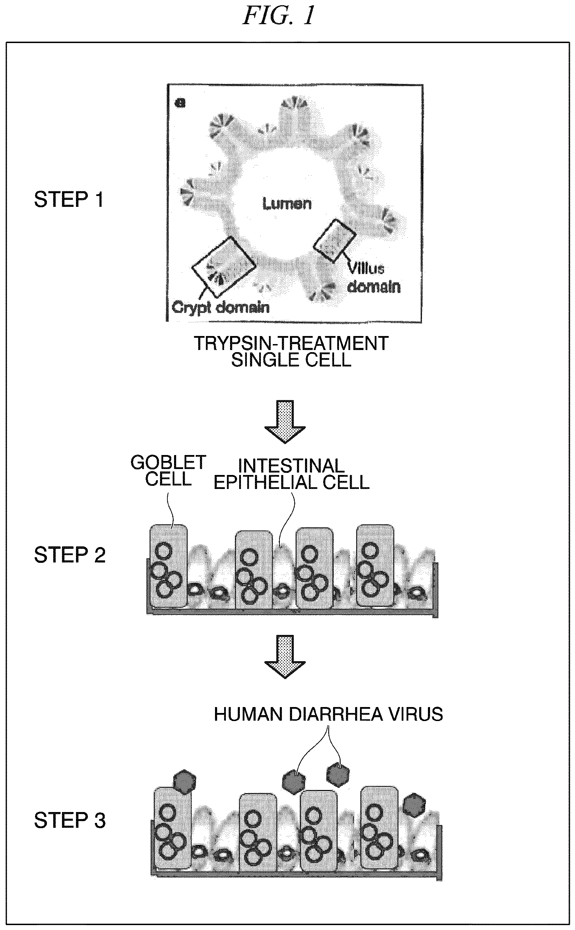

Subsequently, after seeding the cell, the above cell culture medium is added before the cells are dried, and the cells are cultured. A culturing temperature is preferably 30°C or higher and 40°C or lower, and more preferably approximately 37°C. A culture time can be appropriately adjusted depending on the cells to be used. An organoid can be formed approximately 1 to 2 weeks after the start of culture. In addition, in the step 1, with respect to cells which can only be maintained and cultured for 2 to 3 months in the related art, the cells can be maintained and cultured even for a long period of time of 3 months or longer (preferably approximately 10 months). A 3D organoid is obtained by the step 1 (see FIG. 1).

In addition, in the organoid-forming step, culture may also be carried out under a hypoxic condition. It is possible to form an organoid with high efficiency from the human intestinal epithelial stem cell, the human intestinal epithelial cell, the human intestinal epithelial tumor cell, or the tissue containing at least any one of these cells, by carrying out the culture under a hypoxic condition.

In the hypoxic condition in the present embodiment, an oxygen concentration is preferably 0.1% or more 15% or less, more preferably 0.3% or more 10% or less, and still more preferably 0.5% or more 5% or less.

### (Step 2)

The step 2 is a step in which the 3D organoid obtained in the step 1 is dispersed to prepare a single cell, and the single cell is monolayer-cultured on an extracellular matrix to obtain a 2D organoid.

As shown in FIG. 1, in the 3D organoid, a site that is infected with the human diarrhea virus is an inner part of an intestinal tract. Therefore, it is necessary to cut the 3D organoid to expose the inner part.

A method of preparing a single cell by dispersing the 3D organoid is not particularly limited, and examples thereof include a physical method and an enzyme treatment method. However, from the viewpoint of not damaging the cells, the enzyme treatment method is preferable. Examples of the enzyme to be used in the enzyme treatment method include the same as those exemplified in the [Adhesion Step], such as trypsin.

Subsequently, the cell is seeded on an extracellular matrix prepared in the same manner as the [Extracellular Matrix Preparation Step], and allowed to stand. The seeded cells can adhere to the ECM by interacting with a surface structure of the ECM, for example, by interacting with an integrin.

Subsequently, after seeding the cell, the above differentiation medium is added before the cells are dried, and the cells are monolayer-cultured. A 2D organoid is obtained by the step 2 (see FIG. 1).

### (Step 3)

The step 3 is a step in which the 2D organoid obtained in the step 2 is infected with the human diarrhea virus and the infected 2D organoid is cultured (see FIG. 1). Since a cell surface where the 2D organoid contacts with a liquid medium also includes an inner surface of the 3D organoid, infection with the human diarrhea virus is effectively performed.

Examples of the human diarrhea virus include rotavirus, norovirus, sapovirus, astrovirus, intestinal tract adenovirus, parechovirus, and aichivirus, and the norovirus is preferable. As described later in examples, the human diarrhea virus grows by the step 3.

According to the production method of human diarrhea virus of the present embodiment, it is possible to artificially produce the human diarrhea virus in a large amount in vitro by performing growth culture, which can be useful to explain the pathology to human diarrhea virus, to develop an antiviral drug and antiseptic, and to develop a vaccine.

### [Kit]

In an embodiment, the present invention provides a production kit for human diarrhea virus including a cell culture medium containing at least one selected from the group consisting of i) Wnt agonist, ii) at least one selected from the group consisting of an insulin-like growth factor 1 (IGF1), a fibroblast growth factor 2 (FGF2), an epidermal growth factor (EGF), and epiregulin (EREG), iii) a bone morphogenetic factor (bone morphogenetic protein; BMP) inhibitor, iv) a transforming growth factor-β (TGF-β) inhibitor, and v) a γ-selectase inhibitor; wherein the Wnt agonist is a Wnt protein and R-spongin, and wherein the cell culture medium contains the IGF1 and the FGF2.

The kit for the present embodiment can also be said to be a culture kit for a 2D organoid for infection and growth culture of human diarrhea virus including a cell culture medium containing at least one selected from the group consisting of i) Wnt agonist, ii) at least one selected from the group consisting of an insulin-like growth factor 1 (IGF1), a fibroblast growth factor 2 (FGF2), an epidermal growth factor (EGF), and epiregulin (EREG), iii) a bone morphogenetic factor (bone morphogenetic protein; BMP) inhibitor, iv) a transforming growth factor-β (TGF-β) inhibitor, and v) a γ-selectase inhibitor and in which the cell culture medium contains the IGF1 and the FGF2.

In the kit for the present embodiment, the cell culture medium may further contain animal-derived bile. As described later in examples, it is possible to remarkably promote growth of the human diarrhea virus by adding the animal-derived bile to the cell culture medium.

Examples of the animal-derived bile include bovine bile extract and swine bile extract. These bile extracts may also be commercially available bile extracts.

In the kit for the present embodiment, the cell culture medium may also be serum-free.

The kit for the present embodiment may further include a cell culture instrument, an instruction manual, and the like, in addition to the cell culture medium. Examples of the cell culture instrument and the like can include a cell culture plate, but are not limited thereto.

The kit for the present embodiment can be suitably used for a production method of human diarrhea virus. It is possible to produce the human diarrhea virus more simply and in a shorter time by making a reagent used for the production method of human diarrhea virus into a kit.

### [Production Method of Organoid]

In an embodiment, the present invention provides a production method of an organoid, including: a step 1 in which a human intestinal epithelial stem cell, a human intestinal epithelial cell, or a tissue containing at least any of these cells is three-dimensionally cultured on an extracellular matrix to obtain a 3D organoid, and a step 2 in which a single cell is prepared from the 3D organoid obtained in the step 1 by dispersing using a proteolytic enzyme, the single cell is monolayer-cultured on an extracellular matrix to obtain a 2D organoid in which epithelial cells containing differentiated trophoblastic cells and goblet cells and configuring human intestinal lumen have a monolayer structure, in which in culturing of the steps 1 and 2, a cell culture medium containing at least one selected from the group consisting of i) Wnt agonist, ii) at least one selected from the group consisting of an insulin-like growth factor 1 (IGF1), a fibroblast growth factor 2 (FGF2), an epidermal growth factor (EGF), and epiregulin (EREG), iii) a bone morphogenetic factor (bone morphogenetic protein; BMP) inhibitor, iv) a transforming growth factor-β (TGF-β) inhibitor, and v) a γ-selectase inhibitor is used, and in which the cell culture medium contains the IGF1 and the FGF2.

Details of the steps 1 and 2 are the same as the description in [Production Method of Human Diarrhea Virus]. As described later in the Examples, the 2D organoid contains goblet cells, which are Mucin 2-positive cells.

The 2D organoid can be applied to regenerative medicine, fundamental medical research of epithelial cells, screening of drug response, drug discovery research using disease-derived epithelial organoid, and the like.

### [Culture]

In an embodiment, the present invention provides a culture of a 2D organoid for infection and growth culture of human diarrhea virus. The 2D organoid for infection and growth culture of human diarrhea virus is the same as that described above. Here, the "culture" includes a cell culture medium and an extracellular matrix, in addition to the 2D organoid. In the culture of the present embodiment, the cell culture medium may also be serum-free. In addition, the cell culture medium may also contain animal-derived bile. In addition, the extracellular matrix may also be Matrigel (registered trademark).

### [Applications]

As an embodiment, the present invention provides a screening method of a disinfectant of human diarrhea virus, in particular norovirus, a disinfectant drug discovery, identification of an infected cell with the virus and an infection mechanism, a screening of prophylactic or therapeutic drugs for diarrhea symptoms caused by the virus, and means for developing prophylactic or therapeutic drug discovery.

In the screening of a drug response to human diarrhea virus, when using the above 2D organoid, the organoid is cultured, for example, in a multiwell plate such as a 96-well plate or a 384-well plate. A molecule affecting the organoid and/or human virus with which infection occurs is identified using a library of the molecule. Examples of the library include an antibody fragment library, a peptide phage display library, a peptide library (for example, LOPAP (trademark), manufactured by Sigma Aldrich), a lipid library (manufactured by BioMol), a synthetic compound library (for example, LOPAP (trademark), manufactured by Sigma Aldrich), or a natural compound library (Specs, manufactured by TimTec). Further, a gene library may also be used. Examples of the gene library include a cDNA library, an antisense library, a siRNA, or other noncoding RNA libraries. Specific examples of a method include a method in which a culture is exposed to multiple concentrations of cells infected with the human diarrhea virus and human diarrhea virus test agent over a certain period of time to evaluate the culture at the end of the exposure time.

Further, the 2D organoid of the present embodiment can be an alternative to the use of cell lines such as Caco-2 cells in toxicity assays of new candidate drugs or known or novel nutritional supplements.

Hereinafter, the present invention will be described using Examples, but the present invention is not limited to the following examples.

### Examples

### [Experimental Example 1]

### (Preparation of Cell Culture Medium)

First, human recombinant R-spongin 1 (manufactured by R&D systems) was added to a commercially available Advanced DMEM/F-12 medium (manufactured by Thermo Fisher SCIENTIFIC) such that a final concentration was 1 µg/mL. Noggin (manufactured by Peprotech) was added thereto such that a final concentration was 100 ng/mL. A83-01 (manufactured by Tocris) was added thereto such that a final concentration was 500 nM (hereinafter, referred to as "NRA medium").

Further, Wnt3a at a final concentration of 300 ng/mL, IGF1 (manufactured by Biolegend) at a final concentration of 500 ng/mL, FGF2 (manufactured by Peprotech) at a final concentration of 50 ng/mL, EGF (manufactured by Thermo Fisher SCIENTIFIC) at a final concentration of 50 ng/mL, SB 202190 (manufactured by Sigma Aldrich) at a final concentration of 10 µM, and LY 411575 (manufactured by Sigma Aldrich) at a final concentration of 1 µM were added in the following combinations respectively to prepare a medium.
- WNRA+IGF1+FGF2 medium
- WENRAS medium
- ENRA medium
- ENRAL medium

The meanings of abbreviations are shown below.
W; Wnt3A
E; EGF
N; Noggin
R; Rspondin 1
A; A-83-01
S; SB 202190
L; LY 411575

### [Experimental Example 2]

### (Growth of Norovirus using Organoid)

### «Culture of Intestinal Stem Cell»

Based on the ethics research plan approved by the Ethics Committee at the Medical School of Keio University, a part at least 5 cm or farther away from a gastrointestinal tract tumor was collected as a normal mucosa, from normal persons and patients with a gastrointestinal tract tumor, who provided informed consent. An epithelial cell was extracted with EDTA or liberase TH from the collected tissue and embedded in Matrigel (registered trademark).

Matrigel (registered trademark) containing the epithelial cell (hereinafter, referred to as "intestinal stem cell") was seeded in a 48-well plate and cultured. Specifically, the procedure was as follows.

The cultured intestinal stem cells were seeded in a 48-well plate together with 25 µL of Matrigel (registered trademark) (manufactured by BD Bioscience). Each 100 µL of the WNRA+IGF1+FGF2 medium prepared in (1) was added to wells and incubated at 37°C. Medium exchange was performed every 2 days after the culturing and culturing was performed for 7 days to obtain a 3D organoid.

### <<Production of Plate>>

50 µL/well of 2.5% Matrigel (registered trademark) diluted with PBS(-) was added to a 96-well plate, and incubated at 37°C for 1 hour or longer. Thereafter, each well was washed three times with PBS(-) and used for the following cell culture.

### <<Organoid Plate Culture and Norovirus Infection>>

A 2D organoid was formed and infected with norovirus. Specifically, the above organoid was cut using TrypLE (trademark) Express (manufactured by Thermo Fisher SCIENTIFIC) to make a single cell. The single cell was divided into 3 cells, washed with each medium (WENRAS medium (a growth medium), an ENRA medium (a differentiation medium 1), an ENRAL medium (a differentiation medium 2)), and then suspended in each medium containing Y-27632 (Rock inhibitor; manufactured by Wako Pure Chemical Industries, Ltd.) at a final concentration of 10 µM. The cell suspension was inoculated at 1 × 10⁵ cells/well, allowed to stand still for 3 hours or longer and adhered to a plate.

Next, 10% emulsion was incubated for 3 hours in each differentiation medium by adding 5 µL/well of fecal norovirus diluted 10-fold thereto. Thereafter, washing was performed 3 times with PBS(-), and each medium was added at 250 µL/well, and simultaneously 20 µL thereof was sampled (day 0). Thereafter, 20 µL of the medium on day 1, day 2, day 3, and day 6 after infection was sampled (day 1, day 2, day 3, and day 6).

### «Purification of Norovirus RNA»

Viral RNA was purified from the sampled medium by using a high pure viral RNAkit (manufactured by Roche Life Science) to obtain 30 µL of viral RNA suspension.

### <<Measurement of Norovirus Genome by Real-Time qRT-PCR>>

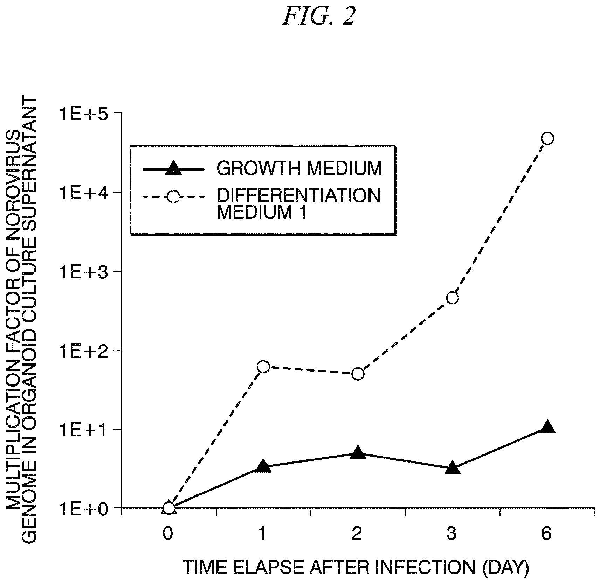

A 10-fold dilution series was prepared from 10⁹ copies/µL of template DNA, and this was used as a standard. A real-time qRT-PCR mix was prepared with compositions shown in Table 1. In addition, qRT-PCR was performed in a cycle shown in Table 2. The results of the qRT-PCR are shown in FIG. 2.

**[Table 1]**

| Component | Volume (µL/1 reaction) |
|---|---|
| Taqman Fast virus 1-step master mix, 4x | 5 |
| Primer C0G2F (CARGARBCNATGTTYAGRTGGATGAG (Sequence No. 1)), 100 pmol/µL | 0.1 |
| Primer C0G2R (TCGACGCCATCTTCATTCACA (Sequence No. 2), 100 pmol/µL | 0.1 |
| Ring2 probe (TP probe-FAM-TGGGAGGGCGATCGCAATCT (Sequence No. 3)-TAMRA), 5 pmol/µL | 0.5 |
| DW with salmon sperm DNA (100 µg/mL) | 9.3 |
| Premix volume | 15 |
| RNA/DNA sample | 5 |
| Total volume | 20 |

**[Table 2]**

| | | | |
|---|---|---|---|
| Reverse transcription | | 50°C | 10 min. |
| Initial denaturation | | 95°C | 30 sec. |
| Amplification (x55 cycle) | Amplification 1 | 95°C | 5 sec. |
| | Amplification 2 | 60°C | 30 sec. |
| Cooling | | 4°C | 30 sec. |

As shown in FIG. 2, a multiplication factor of norovirus genome in organoid culture supernatant in the case of using the differentiation medium 1 was overwhelmingly high, compared to the case of using the growth medium. From this, it was confirmed that the cultured organoid obtained by using the differentiation medium 1 causes infection with human norovirus and growth thereof.

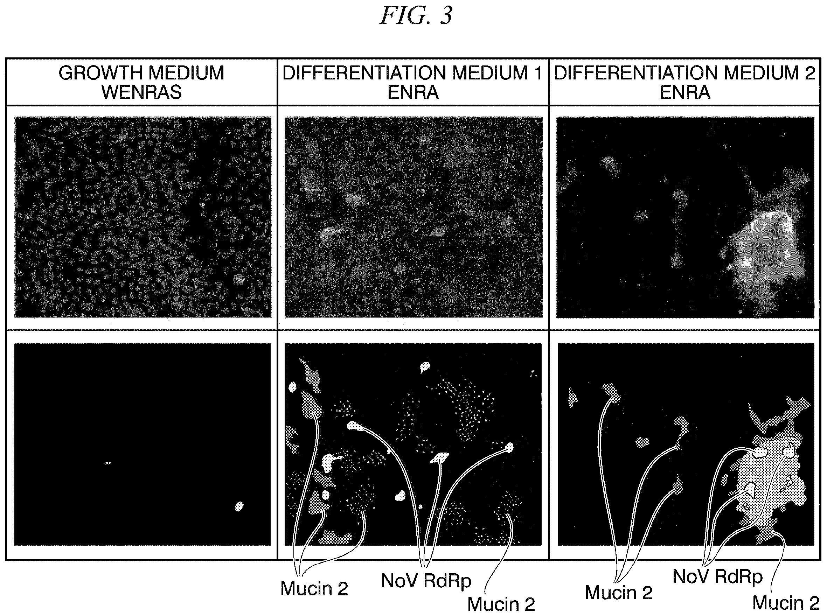

In addition, the organoid cultured in each medium 6 days after the infection was subjected to DAPI staining and immunostaining with an anti-human norovirus RNA-dependent RNA polymerase (RdRp) antibody and an anti-Mucin 2 antibody. Fluorescent stained images of the organoid are shown in FIG. 3.

As shown in the middle and right views of FIG. 3, the expression of the RdRp was confirmed. Accordingly, it was confirmed that an organoid was infected with norovirus and the norovirus was grown. In addition, as shown in the middle and right views of FIG. 3, expression of Mucin 2 was confirmed. Accordingly, it was confirmed that an organoid differentiated into caliciform (goblet) cells.

### [Experimental Example 3]

### (Examination of Extracellular Matrix)

The present inventors found that cell adhesion of the 2D organoid may not be stabilized in some cases.

Therefore, an extracellular matrix was examined. As the extracellular matrix, Collagen I and Matrigel (registered trademark) were used.

### <<Production of Plate>>

50 µL/well of 2.5% Matrigel (registered trademark) diluted with PBS(-) or 10% Collagen I was added to a 96-well plate, and incubated at 37°C for 1 hour or longer. Thereafter, each well was washed three times with PBS(-) and used for the following cell culture. In addition, a 96-well plate without coating was used for comparison.

### «3D Organoid Formation»

As in Experimental Example 2, an intestinal stem cell was cultured to obtain a 3D organoid.

### «2D Organoid Formation»

The obtained organoid was cut using TrypLE (trademark) Express (manufactured by Thermo Fisher SCIENTIFIC) to make a single cell. The single cell was washed with the ENRA medium (differentiation medium 1), and then suspended in the ENRA medium containing Y-27632 (Rock inhibitor; manufactured by Wako Pure Chemical Industries, Ltd.) at a final concentration of 10 µM. The cell suspension was inoculated at 1 × 10⁵ cells/well, and cell adhesion on next day was observed.

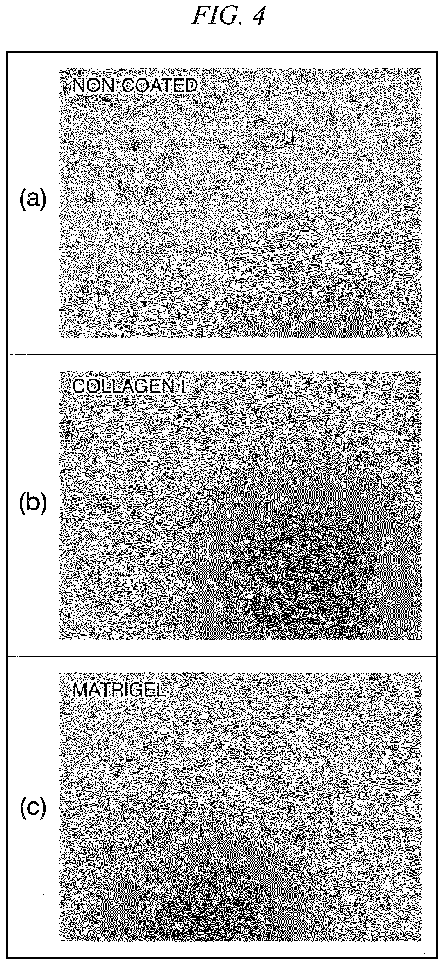

FIGS. 4(a) to 4(c) are optical micrographs of 2D organoids in each condition. FIG. 4(a) shows a result obtained by using a 96-well plate without coating. FIG. 4(b) shows a result obtained by using Collagen I as an extracellular matrix. FIG. 4(c) shows a result obtained by using Matrigel (registered trademark) as an extracellular matrix.

As a result, it became clear that when using Matrigel as an extracellular matrix, adhesion of the 2D organoid to a substrate and a culture substrate becomes favorable.

### [Experimental Example 4]

### (Examination of Cell Culture Medium)

In the growth of norovirus using organoid, animal-derived bile was added to the cell culture medium and an effect thereof was examined. In addition, as a medium of a 3D organoid and a medium of a 2D organoid, the WENRAS medium (growth medium) and the ENRA medium (differentiation medium 1) used in Experimental Example 2 were used in various combinations, and an effect thereof was examined.

### <<Production of Plate>>

50 µL/well of 2.5% Matrigel (registered trademark) diluted with PBS(-) was added to a 96-well plate, and incubated at 37°C for 1 hour or longer. Thereafter, each well was washed three times with PBS(-) and used for the following cell culture.

### «3D Organoid Formation»

As in Experimental Example 2, an intestinal stem cell was cultured to obtain a 3D organoid. At this time, as a medium, the WENRAS medium (growth medium) or the ENRA medium (differentiation medium 1) used in Experimental Example 2 was used.

### «2D Organoid Formation»

The obtained 3D organoid was cut using TrypLE (trademark) Express (manufactured by Thermo Fisher SCIENTIFIC) to make a single cell. The single cell was washed with the WENRAS medium (growth medium) or the ENRA medium (differentiation medium 1), and then suspended in each medium containing Y-27632 (Rock inhibitor; manufactured by Wako Pure Chemical Industries, Ltd.) at a final concentration of 10 µM. In addition, each medium, to which 0.1% porcine bile extract (Bile extract porcine (BEP)) was added or not added, was prepared. The cell suspension was inoculated at 1 × 10⁵ cells/well, allowed to stand still for 3 hours or longer and adhered to a plate.

Combinations of the media used for culturing a 3D organoid and culturing a 2D organoid are shown in Table 3 below. In Table 3, "+" indicates added and "-" indicates not added.

**[Table 3]**

| Group | Porcine bile extract | Medium of 3D organoid | Medium of 2D organoid |
|---|---|---|---|
| 1 | - | Growth medium | Growth medium |
| 2 | + | Growth medium | Growth medium |
| 3 | - | Growth medium | Differentiation medium 1 |
| 4 | + | Growth medium | Differentiation medium 1 |
| 5 | - | Differentiation medium 1 | Growth medium |
| 6 | + | Differentiation medium 1 | Growth medium |
| 7 | - | Differentiation medium 1 | Differentiation medium 1 |
| 8 | + | Differentiation medium 1 | Differentiation medium 1 |

Next, 10% emulsion was incubated for 3 hours in each differentiation medium by adding 5 µL/well of fecal norovirus diluted 10-fold thereto. Thereafter, washing was performed 3 times with PBS(-), and each medium was added at 250 µL/well, and simultaneously 20 µL thereof was sampled (day 0). Thereafter, 20 µL of the medium on day 1, day 2, day 3, and day 6 after infection was sampled (day 1, day 2, day 3, and day 6).

### «Purification of Norovirus RNA»

Viral RNA was purified from the sampled medium by using a high pure viral RNAkit (manufactured by Roche Life Science) to obtain 30 µL of viral RNA suspension.

### <<Measurement of Norovirus Genome by Real-Time qRT-PCR>>

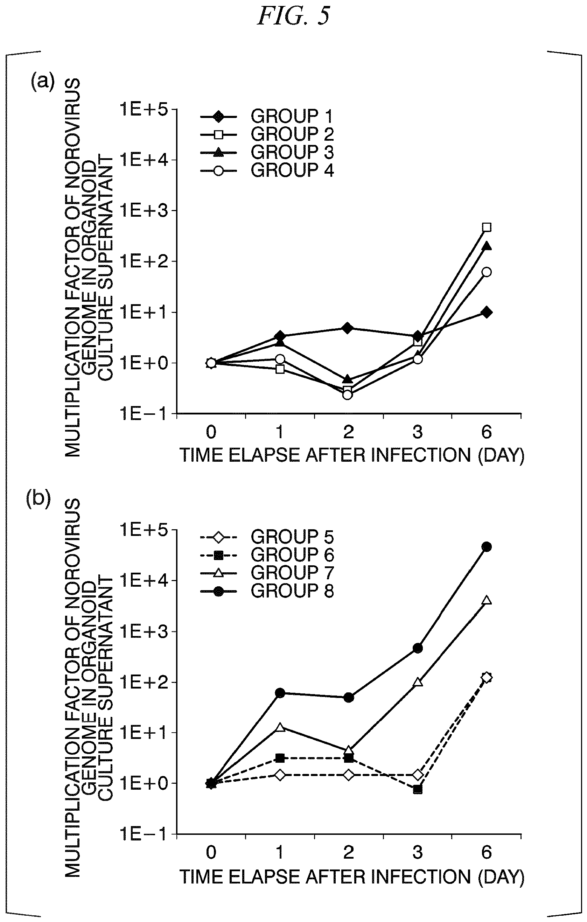

Real-time qRT-PCR was performed in the same manner as in Experimental Example 2 to measure the growth of norovirus. FIGS. 5(a) and 5(b) are graphs showing results of the qRT-PCR. FIG. 5(a) shows results of groups 1 to 4 and FIG. 5(b) shows results of groups 5 to 8.

As a result, it became clear that the growth of norovirus was remarkably high in groups 7 and 8 in which differentiation medium was used for culturing a 3D organoid and culturing a 2D organoid. In addition, it became clear that the growth of norovirus in group 8 containing animal-derived bile in the medium was even more remarkably higher.

### Industrial Applicability

According to the present invention, it is possible to artificially produce human diarrhea virus in a large amount in vitro by performing growth culture, which can be useful to explain the pathology to human diarrhea virus, to develop an antiviral drug and antiseptic, and to develop a vaccine.

## Claims

1. A production method of a 2D organoid for infection and growth culture of human diarrhea virus, comprising:
a step 1 in which a human intestinal epithelial stem cell, a human intestinal epithelial cell, or a tissue containing at least any of these cells is three-dimensionally cultured on an extracellular matrix to obtain a 3D organoid; and
a step 2 in which the 3D organoid obtained in the step 1 is dispersed to prepare a single cell, and the single cell is monolayer-cultured on an extracellular matrix to obtain a 2D organoid in which epithelial cells containing differentiated trophoblastic cells and goblet cells and configuring human intestinal lumen have a monolayer structure,
wherein in culturing of the steps 1 and 2, a cell culture medium containing at least one selected from the group consisting of i) Wnt agonist, ii) at least one selected from the group consisting of an insulin-like growth factor 1 (IGF1), a fibroblast growth factor 2 (FGF2), an epidermal growth factor (EGF), and epiregulin (EREG), iii) a bone morphogenetic factor (bone morphogenetic protein; BMP) inhibitor, iv) a transforming growth factor-β (TGF-β) inhibitor, and v) a γ-selectase inhibitor is used, and
wherein the cell culture medium contains the IGF1 and the FGF2.

2. The production method of a 2D organoid for infection and growth culture of human diarrhea virus according to Claim 1, wherein the Wnt agonist is at least one selected from the group consisting of a Wnt protein, R-spongin, and a GSK-3β inhibitor.

3. The production method of a 2D organoid for infection and growth culture of human diarrhea virus according to Claim 1 or 2, wherein the Wnt protein forms a complex with afamin, which is a stabilizing substance thereof.

4. The production method of a 2D organoid for infection and growth culture of human diarrhea virus according to any one of Claims 1 to 3, wherein the Wnt protein is at least one selected from the group consisting of Wnt1, Wnt2, Wnt2b, Wnt3, Wnt3a, Wnt4, Wnt5a, Wnt5b, Wnt6, Wnt7a, Wnt7b, Wnt8a, Wnt8b, Wnt9a, Wnt9b, Wnt10a, Wnt10b, Wnt11, and Wnt16.

5. The production method of a 2D organoid for infection and growth culture of human diarrhea virus according to any one of Claims 1 to 4, wherein the TGF-β inhibitor is at least one selected from the group consisting of A83-01, SB-431542, SB-505124, SB-525334, SD-208, LY-36494, and SJN-2511.

6. The production method of a 2D organoid for infection and growth culture of human diarrhea virus according to Claim 5, wherein the TGF-β inhibitor is A83-01.

7. The production method of a 2D organoid for infection and growth culture of human diarrhea virus according to any one of Claims 1 to 6, wherein the cell culture medium further contains animal-derived bile.

8. The production method of a 2D organoid for infection and growth culture of human diarrhea virus according to any one of Claims 2 to 7, wherein the R-spongin is at least one selected from the group consisting of R-spongin 1, R-spongin 2, R-spongin 3, and R-spongin 4.

9. The production method of a 2D organoid for infection and growth culture of human diarrhea virus according to any one of Claims 1 to 8, wherein the BMP inhibitor is at least one selected from the group consisting of a chordin-like protein such as noggin, gremlin, chordin, and a chordin domain, follistatin-related protein such as follistatin and a follistatin domain, a DAN-like protein such as DAN and a DAN cysteine-knot domain, sclerostin/SOST, and α-2 macroglobulin.

10. The production method of a 2D organoid for infection and growth culture of human diarrhea virus according to Claim 9, wherein the BMP inhibitor is noggin.

11. The production method of a 2D organoid for infection and growth culture of human diarrhea virus according to any one of Claims 1 to 10, wherein in the steps 1 and 2, the extracellular matrix is Matrigel

12. The production method of a 2D organoid for infection and growth culture of human diarrhea virus according to any one of Claims 1 to 11, which is performed under serum-free.

13. A production method of human diarrhea virus, comprising:
a step 1 in which a human intestinal epithelial stem cell, a human intestinal epithelial cell, or a tissue containing at least any of these cells is three-dimensionally cultured on an extracellular matrix to obtain a 3D organoid; and
a step 2 in which the 3D organoid obtained in the step 1 is dispersed to prepare a single cell, and the single cell is monolayer-cultured on an extracellular matrix to obtain a 2D organoid in which epithelial cells containing differentiated trophoblastic cells and goblet cells and configuring human intestinal lumen have a monolayer structure,
a step 3 in which the 2D organoid is infected with human diarrhea virus and the infected 2D organoid is cultured to perform infection and growth culture of human diarrhea virus,
wherein in culturing of the steps 1 and 2, a cell culture medium containing at least one selected from the group consisting of i) Wnt agonist, ii) at least one selected from the group consisting of an insulin-like growth factor 1 (IGF1), a fibroblast growth factor 2 (FGF2), an epidermal growth factor (EGF), and epiregulin (EREG), iii) a bone morphogenetic factor (bone morphogenetic protein; BMP) inhibitor, iv) a transforming growth factor-β (TGF-β) inhibitor, and v) a γ-selectase inhibitor is used, and
wherein the cell culture medium contains the IGF1 and the FGF2.

14. A culture kit for a 2D organoid for infection and growth culture of human diarrhea virus, comprising:
a cell culture medium containing at least one selected from the group consisting of i) Wnt agonist, ii) at least one selected from the group consisting of an insulin-like growth factor 1 (IGF1), a fibroblast growth factor 2 (FGF2), an epidermal growth factor (EGF), and epiregulin (EREG), iii) a bone morphogenetic factor (bone morphogenetic protein; BMP) inhibitor, iv) a transforming growth factor-β (TGF-β) inhibitor, and v) a γ-selectase inhibitor,
wherein the cell culture medium contains the IGF1 and the FGF2.

15. The culture kit for a 2D organoid for infection and growth culture of human diarrhea virus according to Claim 14, wherein the cell culture medium further contains animal-derived bile.

16. A production kit for human diarrhea virus, comprising:
a cell culture medium containing at least one selected from the group consisting of i) Wnt agonist, ii) at least one selected from the group consisting of an insulin-like growth factor 1 (IGF1), a fibroblast growth factor 2 (FGF2), an epidermal growth factor (EGF), and epiregulin (EREG), iii) a bone morphogenetic factor (bone morphogenetic protein; BMP) inhibitor, iv) a transforming growth factor-β (TGF-β) inhibitor, and v) a γ-selectase inhibitor, wherein the Wnt agonist is a Wnt protein and R-spongin,
wherein the cell culture medium contains the IGF1 and the FGF2.

17. The production kit for human diarrhea virus according to Claim 16, wherein the Wnt protein is at least one selected from the group consisting of Wnt1, Wnt2, Wnt2b, Wnt3, Wnt3a, Wnt4, Wnt5a, Wnt5b, Wnt6, Wnt7a, Wnt7b, Wnt8a, Wnt8b, Wnt9a, Wnt9b, Wnt10a, Wnt10b, Wnt11, and Wnt16.

18. The production kit for human diarrhea virus according to Claim 16 or 17, wherein the R-spongin is at least one selected from the group consisting of R-spongin 1, R-spongin 2, R-spongin 3, and R-spongin 4.

19. The production kit for human diarrhea virus according to any one of Claims 16 to 18, wherein the BMP inhibitor is at least one selected from the group consisting of a chordin-like protein such as noggin, gremlin, chordin, and a chordin domain, follistatin-related protein such as follistatin and a follistatin domain, a DAN-like protein such as DAN and a DAN cysteine-knot domain, sclerostin/SOST, and α-2 macroglobulin.

20. The production kit for human diarrhea virus according to any one of Claims 16 to 19, wherein the BMP inhibitor is noggin.

21. The production kit for human diarrhea virus according to any one of Claims 16 to 20, wherein the Wnt agonist is a Wnt protein and R-spongin.

22. The production kit for human diarrhea virus according to any one of Claims 16 to 21, wherein the TGF-β inhibitor is at least one selected from the group consisting of A83-01, SB-431542, SB-505124, SB-525334, SD-208, LY-36494, and SJN-2511.

23. The production kit for human diarrhea virus according to any one of Claims 16 to 22, wherein the TGF-β inhibitor is A83-01.

24. The production kit for human diarrhea virus according to any one of Claims 16 to 23, wherein the Wnt protein forms a complex with afamin, which is a stabilizing substance thereof.

## Patentansprüche

1. Produktionsverfahren eines 2D-Organoids für eine Infektions- und Wachstumskultur von humanem Diarrhoevirus, umfassend:
einen Schritt 1, in dem eine humane Darmepithel-Stammzelle, eine humane Darmepithelzelle oder ein Gewebe, dass mindestens beliebige dieser Zellen enthält, dreidimensional auf einer extrazellulären Matrix kultiviert wird, um ein 3D-Organoid zu erhalten; und
einen Schritt 2, in dem das im Schritt 1 erhaltene 3D-Organoid verteilt wird, um eine einzelne Zelle herzustellen, und die einzelne Zelle auf einer extrazellulären Matrix Monolayer-kultiviert wird, um ein 2D-Organoid zu erhalten, in dem Epithelzellen, die differenzierte Trophoblastzellen und Becherzellen enthalten und humanes Darmlumen konfigurieren, eine Monolayer-Struktur aufweisen,
wobei im Kultivieren der Schritte 1 und 2 ein Zellkulturmedium, das mindestens einen enthält, der aus der Gruppe bestehend aus i) Wnt-Agonist, ii) mindestens einem, der aus der Gruppe bestehend aus einem insulinähnlichen Wachstumsfaktor 1 (IGF1), einem Fibroblastenwachstumsfaktor 2 (FGF2), einem epidermalen Wachstumsfaktor (EGF) und Epiregulin (EREG) ausgewählt ist, iii) einem Inhibitor von knochenmorphogenetischem Faktor (knochenmorphogenetisches Protein; BMP), iv) einem Inhibitor von transformierendem Wachstumsfaktor-β (TGF-(3) und v) einem γ-Selektase-Inhibitor ausgewählt ist, verwendet wird und
wobei das Zellkulturmedium den IGF1 und den FGF2 enthält.

2. Produktionsverfahren eines 2D-Organoids für eine Infektions- und Wachstumskultur von humanem Diarrhoevirus nach Anspruch 1, wobei der Wnt-Agonist mindestens einer ist, der aus der Gruppe bestehend aus einem Wnt-Protein, R-Spongin und einem GSK-3β-Inhibitor ausgewählt ist.

3. Produktionsverfahren eines 2D-Organoids für eine Infektions- und Wachstumskultur von humanem Diarrhoevirus nach Anspruch 1 oder 2, wobei das Wnt-Protein einen Komplex mit Afamin, das eine Stabilisierungssubstanz davon ist, bildet.

4. Produktionsverfahren eines 2D-Organoids für eine Infektions- und Wachstumskultur von humanem Diarrhoevirus nach einem der Ansprüche 1 bis 3, wobei das Wnt-Protein mindestens eines ist, das aus der Gruppe bestehend aus Wnt1, Wnt2, Wnt2b, Wnt3, Wnt3a, Wnt4, Wnt5a, Wnt5b, Wnt6, Wnt7a, Wnt7b, Wnt8a, Wnt8b, Wnt9a, Wnt9b, Wnt10a, Wnt10b, Wnt11 und Wnt16 ausgewählt ist.

5. Produktionsverfahren eines 2D-Organoids für eine Infektions- und Wachstumskultur von humanem Diarrhoevirus nach einem der Ansprüche 1 bis 4, wobei der TGF-β-Inhibitor mindestens einer ist, der aus der Gruppe bestehend aus A83-01, SB-431542, SB-505124, SB-525334, SD-208, LY-36494 und SJN-2511 ausgewählt ist.

6. Produktionsverfahren eines 2D-Organoids für eine Infektions- und Wachstumskultur von humanem Diarrhoevirus nach Anspruch 5, wobei der TGF-β-Inhibitor A83-01 ist.

7. Produktionsverfahren eines 2D-Organoids für eine Infektions- und Wachstumskultur von humanem Diarrhoevirus nach einem der Ansprüche 1 bis 6, wobei das Zellkulturmedium ferner aus Tieren gewonnene Galle enthält.

8. Produktionsverfahren eines 2D-Organoids für eine Infektions- und Wachstumskultur von humanem Diarrhoevirus nach einem der Ansprüche 2 bis 7, wobei das R-Spongin mindestens eines ist, das aus der Gruppe bestehend aus R-Spongin 1, R-Spongin 2, R-Spongin 3 und R-Spongin 4 ausgewählt ist.

9. Produktionsverfahren eines 2D-Organoids für eine Infektions- und Wachstumskultur von humanem Diarrhoevirus nach einem der Ansprüche 1 bis 8, wobei der BMP-Inhibitor mindestens einer ist, der aus der Gruppe bestehend aus einem chordinähnlichen Protein, wie Noggin, Gremlin, Chordin und eine Chordin-Domäne, follistatinverwandtem Protein, wie Follistatin und eine Follistatin-Domäne, einem DAN-ähnlichen Protein, wie DAN und eine DAN-Cystein-Knot-Domäne, Sclerostin/SOST und α-2-Makroglobulin ausgewählt ist.

10. Produktionsverfahren eines 2D-Organoids für eine Infektions- und Wachstumskultur von humanem Diarrhoevirus nach Anspruch 9, wobei der BMP-Inhibitor Noggin ist.

11. Produktionsverfahren eines 2D-Organoids für eine Infektions- und Wachstumskultur von humanem Diarrhoevirus nach einem der Ansprüche 1 bis 10, wobei die extrazelluläre Matrix in den Schritten 1 und 2 Matrigel ist.

12. Produktionsverfahren eines 2D-Organoids für eine Infektions- und Wachstumskultur von humanem Diarrhoevirus nach einem der Ansprüche 1 bis 11, das serumfrei durchgeführt wird.

13. Produktionsverfahren von humanem Diarrhoevirus, umfassend:
einen Schritt 1, in dem eine humane Darmepithel-Stammzelle, eine humane Darmepithelzelle oder ein Gewebe, das mindestens eine beliebige dieser Zellen enthält, dreidimensional auf einer extrazellulären Matrix kultiviert wird, um ein 3D-Organoid zu erhalten; und
einen Schritt 2, in dem das im Schritt 1 erhaltene 3D-Organoid verteilt wird, um eine einzelne Zelle herzustellen, und die einzelne Zelle auf einer extrazellulären Matrix Monolayer-kultiviert wird, um ein 2D-Organoid zu erhalten, in dem Epithelzellen, die differenzierte Trophoblastzellen und Becherzellen enthalten und humanes Darmlumen konfigurieren, eine Monolayer-Struktur aufweisen,
einen Schritt 3, in dem das 2D-Organoid mit humanem Diarrhoevirus infiziert wird und das infizierte 2D-Organoid kultiviert wird, um eine Infektions- und Wachstumskultur von humanem Diarrhoevirus durchzuführen,
wobei im Kultivieren der Schritte 1 und 2 ein Zellkulturmedium, das mindestens einen enthält, der aus der Gruppe bestehend aus i) Wnt-Agonist, ii) mindestens einem, der aus der Gruppe bestehend aus einem insulinähnlichen Wachstumsfaktor 1 (IGF1), einem Fibroblastenwachstumsfaktor 2 (FGF2), einem epidermalen Wachstumsfaktor (EGF) und Epiregulin (EREG) ausgewählt ist, iii) einem Inhibitor von knochenmorphogenetischem Faktor (knochenmorphogenetisches Protein; BMP), iv) einem Inhibitor von transformierendem Wachstumsfaktor-(3 (TGF-(3) und v) einem γ-Selektase-Inhibitor ausgewählt ist, verwendet wird und
wobei das Zellkulturmedium den IGF1 und den FGF2 enthält.

14. Kulturkit für ein 2D-Organoid für eine Infektions- und Wachstumskultur von humanem Diarrhoevirus, umfassend:
ein Zellkulturmedium, das mindestens einen enthält, der aus der Gruppe bestehend aus i) Wnt-Agonist, ii) mindestens einem, der aus der Gruppe bestehend aus einem insulinähnlichen Wachstumsfaktor 1 (IGF1), einem Fibroblastenwachstumsfaktor 2 (FGF2), einem epidermalen Wachstumsfaktor (EGF) und Epiregulin (EREG) ausgewählt ist, iii) einem Inhibitor von knochenmorphogenetischem Faktor (knochenmorphogenetisches Protein; BMP), iv) einem Inhibitor von transformierendem Wachstumsfaktor-β (TGF-(3) und v) einem γ-Selektase-Inhibitor ausgewählt ist,
wobei das Zellkulturmedium den IGF1 und den FGF2 enthält.

15. Kulturkit für ein 2D-Organoid für eine Infektions- und Wachstumskultur von humanem Diarrhoevirus nach Anspruch 14, wobei das Zellkulturmedium weiterhin aus Tieren gewonnene Galle enthält.

16. Produktionskit für humanes Diarrhoevirus, umfassend:
ein Zellkulturmedium, das mindestens einen enthält, der aus der Gruppe bestehend aus i) Wnt-Agonist, ii) mindestens einem, der aus der Gruppe bestehend aus einem insulinähnlichen Wachstumsfaktor 1 (IGF1), einem Fibroblastenwachstumsfaktor 2 (FGF2), einem epidermalen Wachstumsfaktor (EGF) und Epiregulin (EREG) ausgewählt ist, iii) einem Inhibitor von knochenmorphogenetischem Faktor (knochenmorphogenetisches Protein; BMP), iv) einem Inhibitor von transformierendem Wachstumsfaktor-β (TGF-(3) und v) einem γ-Selektase-Inhibitor ausgewählt ist, wobei der Wnt-Agonist ein Wnt-Protein und R-Spongin ist,
wobei das Zellkulturmedium den IGF1 und den FGF2 enthält.

17. Produktionskit für humanes Diarrhoevirus nach Anspruch 16, wobei das Wnt-Protein mindestens eines ist, das aus der Gruppe bestehend aus Wnt1, Wnt2, Wnt2b, Wnt3, Wnt3a, Wnt4, Wnt5a, Wnt5b, Wnt6, Wnt7a, Wnt7b, Wnt8a, Wnt8b, Wnt9a, Wnt9b, Wnt10a, Wnt10b, Wnt11 und Wnt16 ausgewählt ist.

18. Produktionskit für humanes Diarrhoevirus nach Anspruch 16 oder 17, wobei das R-Spongin mindestens eines ist, das aus der Gruppe bestehend aus R-Spongin 1, R-Spongin 2, R-Spongin 3 und R-Spongin 4 ausgewählt ist.

19. Produktionskit für humanes Diarrhoevirus nach einem der Ansprüche 16 bis 18, wobei der BMP-Inhibitor mindestens einer ist, der aus der Gruppe bestehend aus einem chordinähnlichen Protein, wie Noggin, Gremlin, Chordin und eine Chordin-Domäne, follistatinverwandtem Protein, wie Follistatin und eine Follistatin-Domäne, einem DAN-ähnlichen Protein, wie DAN und eine DAN-Cystein-Knot-Domäne, Sclerostin/SOST und α-2-Makroglobulin ausgewählt ist.

20. Produktionskit für humanes Diarrhoevirus nach einem der Ansprüche 16 bis 19, wobei der BMP-Inhibitor Noggin ist.

21. Produktionskit für humanes Diarrhoevirus nach einem der Ansprüche 16 bis 20, wobei der Wnt-Agonist ein Wnt-Protein und R-Spongin ist.

22. Produktionskit für humanes Diarrhoevirus nach einem der Ansprüche 16 bis 21, wobei der TGF-β-Inhibitor mindestens einer ist, der aus der Gruppe bestehend aus A83-01, SB-431542, SB-505124, SB-525334, SD-208, LY-36494 und SJN-2511 ausgewählt ist.

23. Produktionskit für humanes Diarrhoevirus nach einem der Ansprüche 16 bis 22, wobei der TGF-β-Inhibitor A83-01 ist.

24. Produktionskit für humanes Diarrhoevirus nach einem der Ansprüche 16 bis 23, wobei das Wnt-Protein einen Komplex mit Afamin, das eine Stabilisierungssubstanz davon ist, bildet.

## Revendications

1. Procédé de production d'un organoïde 2D pour une culture infectieuse et de croissance du virus de la diarrhée humaine, comprenant :
une étape 1 dans laquelle une cellule souche épithéliale intestinale humaine, une cellule épithéliale intestinale humaine ou un tissu contenant au moins l'une quelconque de ces cellules est mis en culture tridimensionnellement sur une matrice extracellulaire pour obtenir un organoïde 3D ; et
une étape 2 dans laquelle l'organoïde 3D obtenu à l'étape 1 est dispersé pour préparer une unique cellule, et l'unique cellule est mise en culture monocouche sur une matrice extracellulaire pour obtenir un organoïde 2D dans lequel des cellules épithéliales contenant des cellules trophoblastiques et des cellules caliciformes différenciées et configurant le lumen intestinal humain présentent une structure monocouche,
dans lequel, au niveau de la mise en culture des étapes 1 et 2, un milieu de culture de cellules contenant au moins l'un sélectionné parmi le groupe constitué par i) un agoniste de Wnt, ii) au moins l'un sélectionné parmi le groupe constitué par un facteur de croissance insulinoïde 1 (IGF1), un facteur de croissance fibroblastique 2 (FGF2), un facteur de croissance épidermique (EGF) et de l'épiréguline (EREG), iii) un inhibiteur de facteur morphogénétique osseux (protéine morphogénétique osseuse ; BMP), iv) un inhibiteur de facteur de croissance transformant β (TGF-β) et v) un inhibiteur de sélectase γ est utilisé, et
dans lequel le milieu de culture de cellules contient l'IGF1 et le FGF2.

2. Procédé de production d'un organoïde 2D pour une culture infectieuse et de croissance du virus de la diarrhée humaine selon la revendication 1, dans lequel l'agoniste de Wnt est au moins l'un sélectionné parmi le groupe constitué par une protéine Wnt, la spongine R et un inhibiteur de GSK-3β.

3. Procédé de production d'un organoïde 2D pour une culture infectieuse et de croissance du virus de la diarrhée humaine selon la revendication 1 ou 2, dans lequel la protéine Wnt forme un complexe avec l'afamine, qui est une substance de stabilisation de celle-ci.

4. Procédé de production d'un organoïde 2D pour une culture infectieuse et de croissance du virus de la diarrhée humaine selon l'une quelconque des revendications 1 à 3, dans lequel la protéine Wnt est au moins l'une sélectionnée parmi le groupe constitué par Wnt1, Wnt2, Wnt2b, Wnt3, Wnt3a, Wnt4, Wnt5a, Wnt5b, Wnt6, Wnt7a, Wnt7b, Wnt8a, Wnt8b, Wnt9a, Wnt9b, Wnt10a, Wnt10b, Wnt11 et Wnt16.

5. Procédé de production d'un organoïde 2D pour une culture infectieuse et de croissance du virus de la diarrhée humaine selon l'une quelconque des revendications 1 à 4, dans lequel l'inhibiteur de TGF-β est au moins l'un sélectionné parmi le groupe constitué par A83-01, SB-431542, SB-505124, SB-525334, SD-208, LY-36494 et SJN-2511.

6. Procédé de production d'un organoïde 2D pour une culture infectieuse et de croissance du virus de la diarrhée humaine selon la revendication 5, dans lequel l'inhibiteur de TGF-β est A83-01.

7. Procédé de production d'un organoïde 2D pour une culture infectieuse et de croissance du virus de la diarrhée humaine selon l'une quelconque des revendications 1 à 6, dans lequel le milieu de culture de cellules contient en outre de la bile d'origine animale.

8. Procédé de production d'un organoïde 2D pour une culture infectieuse et de croissance du virus de la diarrhée humaine selon l'une quelconque des revendications 2 à 7, dans lequel la spongine R est au moins l'une sélectionnée parmi le groupe constitué par la spongine R 1, la spongine R 2, la spongine R 3 et la spongine R 4.

9. Procédé de production d'un organoïde 2D pour une culture infectieuse et de croissance du virus de la diarrhée humaine selon l'une quelconque des revendications 1 à 8, dans lequel l'inhibiteur de BMP est au moins l'un sélectionné parmi le groupe constitué par une protéine chordinique telle que la noggine, la gremline, la chordine et un domaine chordine, une protéine liée à la follistatine telle que la follistatine et un domaine follistatine, une protéine du type DAN telle que DAN et un domaine de noeud de cystéine DAN, la sclérostine/SOST et la macroglobuline α-2.

10. Procédé de production d'un organoïde 2D pour une culture infectieuse et de croissance du virus de la diarrhée humaine selon la revendication 9, dans lequel l'inhibiteur de BMP est la noggine.

11. Procédé de production d'un organoïde 2D pour une culture infectieuse et de croissance du virus de la diarrhée humaine selon l'une quelconque des revendications 1 à 10, dans lequel, au niveau des étapes 1 et 2, la matrice extracellulaire est du Matrigel.

12. Procédé de production d'un organoïde 2D pour une culture infectieuse et de croissance du virus de la diarrhée humaine selon l'une quelconque des revendications 1 à 11, lequel est réalisé sans sérum.

13. Procédé de production du virus de la diarrhée humaine, comprenant :
une étape 1 dans laquelle une cellule souche épithéliale intestinale humaine, une cellule épithéliale intestinale humaine ou un tissu contenant au moins l'une quelconque de ces cellules est mis en culture tridimensionnellement sur une matrice extracellulaire pour obtenir un organoïde 3D ; et
une étape 2 dans laquelle l'organoïde 3D obtenu au niveau de l'étape 1 est dispersé pour préparer une unique cellule, et l'unique cellule est mise en culture monocouche sur une matrice extracellulaire pour obtenir un organoïde 2D dans lequel des cellules épithéliales contenant des cellules trophoblastiques et des cellules caliciformes différenciées et configurant le lumen intestinal humain présentent une structure monocouche ;
une étape 3 dans laquelle l'organoïde 2D est infecté avec le virus de la diarrhée humaine et l'organoïde 2D infecté est mis en culture pour réaliser une culture infectieuse et de croissance du virus de la diarrhée humaine,
dans lequel, au niveau de la mise en culture des étapes 1 et 2, un milieu de culture de cellules contenant au moins l'un sélectionné parmi le groupe constitué par i) un agoniste de Wnt, ii) au moins l'un sélectionné parmi le groupe constitué par un facteur de croissance insulinoïde 1 (IGF1), un facteur de croissance fibroblastique 2 (FGF2), un facteur de croissance épidermique (EGF) et de l'épiréguline (EREG), iii) un inhibiteur de facteur morphogénétique osseux (protéine morphogénétique osseuse ; BMP), iv) un inhibiteur de facteur de croissance transformant β (TGF-β) et v) un inhibiteur de sélectase γ est utilisé, et
dans lequel le milieu de culture de cellules contient l'IGF1 et le FGF2.

14. Kit de culture pour un organoïde 2D pour une culture infectieuse et de croissance du virus de la diarrhée humaine, comprenant :
un milieu de culture de cellules contenant au moins l'un sélectionné parmi le groupe constitué par i) un agoniste de Wnt, ii) au moins l'un sélectionné parmi le groupe constitué par un facteur de croissance insulinoïde 1 (IGF1), un facteur de croissance fibroblastique 2 (FGF2), un facteur de croissance épidermique (EGF) et de l'épiréguline (EREG), iii) un inhibiteur de facteur morphogénétique osseux (protéine morphogénétique osseuse ; BMP), iv) un inhibiteur de facteur de croissance transformant β (TGF-β) et v) un inhibiteur de sélectase γ,
dans lequel le milieu de culture de cellules contient l'IGF1 et le FGF2.

15. Kit de culture pour un organoïde 2D pour une culture infectieuse et de croissance du virus de la diarrhée humaine selon la revendication 14, dans lequel le milieu de culture de cellules contient en outre de la bile d'origine animale.

16. Kit de production pour le virus de la diarrhée humaine, comprenant :
un milieu de culture de cellules contenant au moins l'un sélectionné parmi le groupe constitué par i) un agoniste de Wnt, ii) au moins l'un sélectionné parmi le groupe constitué par un facteur de croissance insulinoïde 1 (IGF1), un facteur de croissance fibroblastique 2 (FGF2), un facteur de croissance épidermique (EGF) et de l'épiréguline (EREG), iii) un inhibiteur de facteur morphogénétique osseux (protéine morphogénétique osseuse ; BMP), iv) un inhibiteur de facteur de croissance transformant β (TGF-β) et v) un inhibiteur de sélectase γ dans lequel l'agoniste de Wnt est une protéine Wnt et la spongine R,
dans lequel le milieu de culture de cellules contient l'IGF1 et le FGF2.

17. Kit de production pour le virus de la diarrhée humaine selon la revendication 16, dans lequel la protéine Wnt est au moins l'une sélectionnée parmi le groupe constitué par Wnt1, Wnt2, Wnt2b, Wnt3, Wnt3a, Wnt4, Wnt5a, Wnt5b, Wnt6, Wnt7a, Wnt7b, Wnt8a, Wnt8b, Wnt9a, Wnt9b, Wnt10a, Wnt10b, Wnt11 et Wnt16.

18. Kit de production pour le virus de la diarrhée humaine selon la revendication 16 ou 17, dans lequel la spongine R est au moins l'une sélectionnée parmi le groupe constitué par la spongine R 1, la spongine R 2, la spongine R 3 et la spongine R 4.

19. Kit de production pour le virus de la diarrhée humaine selon l'une quelconque des revendications 16 à 18, dans lequel l'inhibiteur de BMP est au moins l'un sélectionné parmi le groupe constitué par une protéine chordinique telle que la noggine, la gremline, la chordine et un domaine chordine, une protéine liée à la follistatine telle que la follistatine et un domaine follistatine, une protéine du type DAN telle que DAN et un domaine de noeud de cystéine DAN, la sclérostine/SOST et la macroglobuline α-2.

20. Kit de production pour le virus de la diarrhée humaine selon l'une quelconque des revendications 16 à 19, dans lequel l'inhibiteur de BMP est la noggine.

21. Kit de production pour le virus de la diarrhée humaine selon l'une quelconque des revendications 16 à 20, dans lequel l'agoniste de Wnt est une protéine Wnt et la spongine R.

22. Kit de production pour le virus de la diarrhée humaine selon l'une quelconque des revendications 16 à 21, dans lequel l'inhibiteur de TGF-β est au moins l'un sélectionné parmi le groupe constitué par A83-01, SB-431542, SB-505124, SB-525334, SD-208, LY-36494 et SJN-2511.

23. Kit de production pour le virus de la diarrhée humaine selon l'une quelconque des revendications 16 à 22, dans lequel l'inhibiteur de TGF-β est A83-01.

24. Kit de production pour le virus de la diarrhée humaine selon l'une quelconque des revendications 16 à 23, dans lequel la protéine Wnt forme un complexe avec l'afamine, qui est une substance de stabilisation de celle-ci.
